# EUROPEAN PATENT SPECIFICATION

(11) **EP 4 099 999 B1**
(45) Date of publication and mention of the grant of the patent: **02.04.2025**
(21) Application number: 21750246.7
(22) Date of filing: 03.02.2021
(51) Int. Cl.: A61K 9/08, A61K 31/337, A61K 31/4525, A61K 31/496, A61K 47/10, A61K 47/12, A61K 47/42

(54) **FORMULATIONS OF DOCETAXEL**
DOCETAXEL-FORMULIERUNGEN
FORMULATIONS DE DOCÉTAXEL

(30) Priority: 04.02.2020 US 202062970055 P; 03.03.2020 US 202062984465 P; 28.01.2021 US 202163142811 P
(43) Date of publication of application: 14.12.2022
(73) Proprietor: Zhuhai Beihai Biotech Co., Ltd., Jinwan, Zhuhai, Guangdong 519085 (CN)
(72) Inventor: SUN, Qun, Princeton, NJ 08540 (US)
(74) Representative: Fish & Richardson P.C.
(86) International application number: PCT/US2021/016375
(87) International publication number: WO 2021/158632

(56) References cited:
- WO-A1-2016/187147
- WO-A1-2019/200084
- US-A1- 2010 197 944
- US-A1- 2011 166 214
- US-B2- 10 500 285
- US-B2- 7 771 751
- US-B2- 8 728 528
- US-B2- 9 782 342

## Description

### CROSS-REFERENCE TO RELATED APPLICATIONS

This application claims priority to: U.S. Patent Application No. 62/970,055, filed February 4, 2020; U.S. Patent Application No. 62/984,465, filed March 3, 2020; and U.S. Patent Application Serial No. 63/142,811, filed January 28, 2021.

### TECHNICAL FIELD

This document relates to formulations for the treatment of proliferative diseases, more particularly to formulations comprising docetaxel, or a pharmaceutically acceptable salt thereof, and human serum albumin, and more particularly to formulations comprising docetaxel, or a pharmaceutically acceptable salt thereof, human serum albumin, and ethanol.

### BACKGROUND

Many drugs for parenteral use are insoluble in water, and are thus formulated with solubilizing agents, surfactants, solvents, and/or emulsifiers that are irritating, allergenic, or toxic when administered to patients. *See, e.g.,* Briggs et al., Anesthesis 37, 1099 (1982), and Waugh et al., Am. J. Hasp. Pharmacists, 48, 1520 (1991)). Further, many of these drugs, especially those administered intravenously, cause undesirable side effects such as venous irritation, phlebitis, burning and pain on injection, venous thrombosis, extravasation, and other administration related side effects. Additionally, often free drugs present in formulations induce pain or irritation upon administration.

Taxanes play an important role in the treatment of various solid tumors. As a secondgeneration semi-synthetic taxane derivative, docetaxel is about twice as potent as paclitaxel in inhibiting microtubule depolymerization, and has the unique ability to alter certain classes of microtubules, which differs from most spindle poisons currently used in clinic. However, docetaxel has very poor water solubility. The clinical intravenous administration of commercially available docetaxel (Taxotere^{®}) is formulated in a highly concentrated solution containing 40 mg docetaxel and 1040 mg Polysorbate 80 per mL. This concentrated solution must be carefully diluted with solvent containing 13% ethanol in saline before administration, and must be used within 4 hours due to its limited stability. These attributes limit the administration of docetaxel. Further, it has been reported that docetaxel administration is associated with the occurrence of unpredictable (acute) hypersensitivity reactions and cumulative fluid retention. *See, e.g.,* Trudeau ME et al., J Clin Oncol 1996; 14:422-8, Piccart MJ et al., J Natl Cancer Inst 1995; 87:676-81, Bruno R et al., J Clin Oncol 1998; 16:187-96. These side-effects have been attributed, in part, to the presence of polysorbate 80.

US 2005/0282734 describes complexes of paclitaxel and albumin. Successful formulations described in this document require acidic pH. WO 2014/121033 describes complexes of camptothecin and albumin. US 2012/0076862 describes nanoparticles of taxane and albumin. US 2010/0076008 describes paclitaxel non-covalently bound to human serum albumin. WO 2016/187147 describes complexes and compositions of docetaxel and human serum albumin. WO 2018/081520 describes neutral pH compositions of docetaxel and human serum albumin. WO 2018/204386 describes formulations and compositions of docetaxel and human serum albumin. WO 2019/200084 describes formulations and compositions of docetaxel, human serum albumin, and amino acids.

There is a further need in the art for formulations of docetaxel which have better safety profile and are more convenient to use in clinic. The compositions and methods described in the present application help meet this need.

### SUMMARY

The current marketed formulation of Taxotere comprises either a one-vial formulation or a two-vial formulation (injection and diluent). The one-vial formulation contains a solution of docetaxel in polysorbate 80 and ethanol. The two-vial formulation contains a solution of docetaxel in polysorbate 80 and a solvent vial containing an aqueous solution of ethanol.

The two-vial formulation requires two dilutions prior to admission to the patient. In the two-vial formulation, the drug vial has prior to use to be reconstituted with the solvent vial making sure that polysorbate 80 is properly reconstituted without significant foaming. The reconstituted solution is further diluted by injection of the appropriate amount of the solution into an infusion bag. The one-vial formulation contains the drug solution in a single vial with known concentration. The drug solution is withdrawn from the vial and injected into infusion bag prior to use. The vial does not required to be reconstituted and homogenized prior to use. Thus, for the one-vial formulation there is no risk of forming and more convenient to use.

However, both two-vial and one-vial formulation of the current marketed Taxotere contain polysorbate 80, which could cause severe hypersensitivity reactions to some patients. It would be highly desirable to develop alternative docetaxel formulations which do not contain polysorbate 80 and yet are convenient to use in clinic.

The applicant has now surprisingly found that by adding a solution of human serum albumin into an infusion bag or bottle with a parenterally acceptable vehicle (e.g., a normal saline solution, a dextrose solution, etc.) prior to injecting the drug solution into the infusion bag, there is no need to use polysorbate 80 in the docetaxel formulation of the present disclosure, which can be used conveniently in clinic similar to the way the current marketed one-vial formulation of Taxotere has been used.

In present invention, provided herein is a pharmaceutical formulation of docetaxel comprising two compositions that are mixed prior to being infused or administered to patients, that comprises: (a) a first liquid composition comprising docetaxel, or a pharmaceutically acceptable salt thereof, ethanol, and an organic acid, wherein the organic acid is not aspartic acid or glutamic acid, and (b) a second aqueous composition comprising human serum albumin and a parenterally acceptable vehicle, wherein the pharmaceutical formulation does not contain Polysorbate 80, and wherein the two compositions are mixed in less than 24 hours prior to being infused or administered to patients.

In some embodiments, the mixing of the two compositions is done in an infusion bag or bottle. In some embodiments, the two compositions are mixed in less than 8 hours prior to being infused or administered to patients. In some embodiments, the two compositions are mixed in less than 4 hours prior to being infused or administered to patients. In some embodiments, the two compositions are mixed in less than 1 hour prior to being infused or administered to patients. In some embodiments, the mixing is done in a infusion bag or bottle. In some embodiments, the second aqueous composition is contained in an infusion bag or bottle. In some embodiments, the first liquid composition is injected into a infusion bag or bottle which contains the second aqueous composition.

In some embodiments, the said pharmaceutical formulation does not comprise a lipid (e.g. soybean oil). In some embodiments, the first liquid composition does not comprise a lipid (e.g. soybean oil). In some embodiments, the lipid is soybean oil.

In some embodiments, the said pharmaceutical formulation does not comprise a surfactant. In some embodiments, the first liquid composition does not comprise a surfactant. In some embodiments, the second aqueous composition does not comprise a surfactant.

In some embodiments, the first liquid composition comprises docetaxel, or a pharmaceutically acceptable salt thereof, ethanol, and other organic solvents (e.g. propylene glycol, polyethylene glycol 300, etc.). In some embodiments, the first liquid composition comprises docetaxel, ethanol, and propylene glycol. In some embodiments, the first liquid composition comprises docetaxel, ethanol, and polyethylene glycol 300. In some embodiments, the first liquid composition further comprises an acid. In some embodiments, the first liquid composition comprises docetaxel, ethanol, and an organic acid. The most preferred acid for use in accordance with the present invention is citric acid. In some embodiments, the first liquid composition comprises docetaxel, or a pharmaceutically acceptable salt thereof, ethanol, and citric acid. In some embodiments, the first liquid composition comprises docetaxel, or a pharmaceutically acceptable salt thereof, ethanol, propylene glycol, and citric acid. In some embodiments, the first liquid composition is an ethanol solution containing docetaxel, or a pharmaceutically acceptable salt thereof. In some embodiments, the first liquid composition is an ethanol solution containing docetaxel, or a pharmaceutically acceptable salt thereof, and citric acid. In some embodiments, the first liquid composition is an ethanol/propylene glycol solution containing docetaxel, or a pharmaceutically acceptable salt thereof, and citric acid. In some embodiments, the weight ratio of docetaxel, or a pharmaceutically acceptable salt thereof, and citric acid, in the first liquid composition is from about 5000:1 to about 1:1. In some embodiments, the weight ratio of docetaxel, or a pharmaceutically acceptable salt thereof, and citric acid, in the first liquid composition is from about 2000:1 to about 1:1. In some embodiments, the weight ratio of docetaxel, or a pharmaceutically acceptable salt thereof, and citric acid, in the first liquid composition is from about 1000:1 to about 1:1. In some embodiments, the weight ratio of docetaxel, or a pharmaceutically acceptable salt thereof, and citric acid in the first liquid composition is from about 500:1 to about 1:1. In some embodiments, the weight ratio of docetaxel, or a pharmaceutically acceptable salt thereof, and citric acid, in the first liquid composition is from about 5000: 1 to about 5:1. In some embodiments, the weight ratio of docetaxel, or a pharmaceutically acceptable salt thereof, and citric acid, in the first liquid composition is from about 2000:1 to about 5:1. In some embodiments, the weight ratio of docetaxel, or a pharmaceutically acceptable salt thereof, and citric acid, in the first liquid composition is from about 1000:1 to about 5:1. In some embodiments, the weight ratio of docetaxel, or a pharmaceutically acceptable salt thereof, and citric acid, in the first liquid composition is from about 500:1 to about 5:1. In some embodiments, the weight ratio of docetaxel, or a pharmaceutically acceptable salt thereof, and citric acid, in the first liquid composition is from about 5000:1 to about 10:1. In some embodiments, the weight ratio of docetaxel, or a pharmaceutically acceptable salt thereof, and citric acid, in the first liquid composition is from about 2000:1 to about 10:1. In some embodiments, the weight ratio of docetaxel, or a pharmaceutically acceptable salt thereof, and citric acid, in the first liquid composition is from about 1000:1 to about 10:1. In some embodiments, the weight ratio of docetaxel, or a pharmaceutically acceptable salt thereof, and citric acid, in the first liquid composition is from about 500:1 to about 10:1. In some embodiments, the weight ratio of docetaxel, or a pharmaceutically acceptable salt thereof, and citric acid, in the first liquid composition is from about 2000:1 to about 100:1. In some embodiments, the weight ratio of docetaxel, or a pharmaceutically acceptable salt thereof, and citric acid, in the first liquid composition is from about 1000:1 to about 100:1. In some embodiments, the weight ratio of docetaxel, or a pharmaceutically acceptable salt thereof, and citric acid, in the first liquid composition is from about 500:1 to about 100:1. In some embodiments, the weight ratio of docetaxel, or a pharmaceutically acceptable salt thereof, and citric acid, in the first liquid composition is from about 5:1 to about 20:1. In some embodiments, the weight ratio of docetaxel, or a pharmaceutically acceptable salt thereof, and citric acid, in the first liquid composition is about 5:1, about 10:1, about 15:1, or about 20:1. In some embodiments, the concentration of citric acid in the first liquid composition is from about 0.005 mg/ml to about 10 mg/ml. In some embodiments, the concentration of citric acid in the first liquid composition is from about 0.01 mg/ml to about 2 mg/ml. In some embodiments, the concentration of citric acid in the first liquid composition is from about 0.01 mg/ml to about 1 mg/ml. In some embodiments, the concentration of citric acid in the first liquid composition is from about 0.02 mg/ml to about 0.5 mg/ml. In some embodiments, the concentration of citric acid in the first liquid composition is from about 0.02 mg/ml to about 0.2 mg/ml.

In some embodiments, the first liquid composition comprises docetaxel, or a pharmaceutically acceptable salt thereof, and a solvent including ethanol, wherein docetaxel, or a pharmaceutically acceptable salt thereof, is dissolved in the solvent. In some embodiments, the first liquid composition comprises docetaxel, or a pharmaceutically acceptable salt thereof, an acid, and a solvent including ethanol, wherein docetaxel, or a pharmaceutically acceptable salt thereof, and the acid are dissolved in the solvent. In some embodiments, the first liquid composition comprises docetaxel, or a pharmaceutically acceptable salt thereof, an organic acid, and a solvent including ethanol, wherein docetaxel, or a pharmaceutically acceptable salt thereof, and the organic acid are dissolved in the solvent. In some embodiments, the first liquid composition comprises docetaxel, or a pharmaceutically acceptable salt thereof, citric acid, and a solvent including ethanol, wherein docetaxel, or a pharmaceutically acceptable salt thereof, and citric acid are dissolved in the solvent. In some embodiments, the first liquid composition comprises docetaxel, or a pharmaceutically acceptable salt thereof, and ethanol, wherein docetaxel, or a pharmaceutically acceptable salt thereof, is dissolved in ethanol. In some embodiments, the first liquid composition comprises docetaxel, or a pharmaceutically acceptable salt thereof, citric acid, and ethanol, wherein docetaxel, or a pharmaceutically acceptable salt thereof, and citric acid are dissolved in ethanol. In some embodiments, the first liquid composition comprises docetaxel, or a pharmaceutically acceptable salt thereof, and dehydrated ethanol, wherein docetaxel, or a pharmaceutically acceptable salt thereof, is dissolved in dehydrated ethanol. In some embodiments, the first liquid composition comprises docetaxel, or a pharmaceutically acceptable salt thereof, citric acid, and dehydrated ethanol, wherein docetaxel, or a pharmaceutically acceptable salt thereof, and citric acid are dissolved in dehydrated ethanol.

In some embodiments, the first liquid composition is prepared by dissolving docetaxel, or a pharmaceutically acceptable salt thereof, in an alcohol (e.g., ethanol). In some embodiments, the first liquid composition is prepared by dissolving docetaxel, or a pharmaceutically acceptable salt thereof, and citric acid in an alcohol (e.g., ethanol). In some embodiments, the first liquid composition is prepared by dissolving docetaxel, or a pharmaceutically acceptable salt thereof, in ethanol and propylene glycol. In some embodiments, the first liquid composition is prepared by dissolving docetaxel, or a pharmaceutically acceptable salt thereof, and citric acid in ethanol and propylene glycol. In some embodiments, concentration of docetaxel in the alcohol (e.g., ethanol and/or propylene glycol) is from about 10 mg/ml to about 30 mg/ml. In some embodiments, concentration of docetaxel in the alcohol (e.g., ethanol and/or propylene glycol) is about 10 mg/ml, about 20 mg/ml, or about 30 mg/ml.

In some embodiments, the second aqueous composition is prepared by adding a human serum albumin solution into a parenterally acceptable vehicle. In some embodiments, the second aqueous composition is prepared by adding a human serum albumin solution into an infusion bag or bottle which contains a parenterally acceptable vehicle. In some embodiments, the second aqueous composition is prepared by adding a human serum albumin solution into a normal saline solution or dextrose solution. In some embodiments, the second aqueous composition is prepared by adding a human serum albumin solution into an infusion bag or bottle which contains a normal saline solution or dextrose solution. In some embodiments, the second aqueous composition is prepared by adding a solution of human serum albumin for infusion into a normal saline solution or dextrose solution. In some embodiments, the second aqueous composition is prepared by adding a solution of human serum albumin for infusion into a normal saline solution or dextrose solution. In some embodiments, the second aqueous composition is prepared by adding a 20% solution of human serum albumin for infusion (w/v) into a normal saline solution or dextrose solution. In some embodiments, the second aqueous composition is prepared by adding a 25% solution of human serum albumin for infusion (w/v) into a normal saline solution or dextrose solution. In some embodiments, the second aqueous composition is prepared by adding a 5% solution of human serum albumin for infusion (w/v) into a normal saline solution or dextrose solution. In some embodiments, the second aqueous composition is prepared by adding a solution of human serum albumin for infusion into an infusion bag or bottle which contains a normal saline solution or dextrose solution. In some embodiments, the second aqueous composition is prepared by adding a solution of human serum albumin for infusion into an infusion bag or bottle which contains a normal saline solution or dextrose solution. In some embodiments, the second aqueous composition is prepared by adding a 20% solution of human serum albumin for infusion (w/v) into an infusion bag or bottle which contains a normal saline solution or dextrose solution. In some embodiments, the second aqueous composition is prepared by adding a 25% solution of human serum albumin for infusion (w/v) into an infusion bag or bottle which contains a normal saline solution or dextrose solution. In some embodiments, the second aqueous composition is prepared by adding a 5% solution of human serum albumin for infusion (w/v) into an infusion bag or bottle which contains a normal saline solution or dextrose solution. In some embodiments, the concentration of human serum albumin in the second aqueous composition is from about 0.1% to about 20% (w/v). In some embodiments, the concentration of human serum albumin in the second aqueous composition is from about 0.5% to about 10% (w/v). In some embodiments, the concentration of human serum albumin in the second aqueous composition is from 0.5% to 5% (w/v). In some embodiments, the concentration of human serum albumin in the second aqueous composition is from 1% to 3% (w/v). In some embodiments, the second aqueous composition comprises from about 1 g to about 50 g of human serum albumin. In some embodiments, the second aqueous composition comprises from about 2 g to about 20 g of human serum albumin.

Also, provided herein is a transparent parenteral infusion composition of docetaxel, or a pharmaceutically acceptable salt thereof, free of precipitates, the composition comprising human serum albumin , an organic acid, wherein the organic acid is not aspartic acid or glutamic acid, and said docetaxel, or a pharmaceutically acceptable salt thereof, in a concentration of docetaxel, from about 0.05 mg/ml to about 1 mg/ml in a parenterally acceptable vehicle, wherein the said parenteral infusion solution is obtained by injecting a first liquid composition comprising docetaxel, or a pharmaceutically acceptable salt thereof, the organic acid, and ethanol into an infusion bag or bottle containing a second aqueous composition comprising human serum albumin in a parenterally acceptable vehicle, wherein mixing or agitation is not needed in the injection process, and wherein the said docetaxel infusion solution does not contain Polysorbate 80. In some embodiments, a parenterally acceptable vehicle is a normal saline solution or dextrose solution.

In some embodiments, the said parenteral infusion composition of docetaxel, or a pharmaceutically acceptable salt thereof, does not comprise a lipid (e.g. soybean oil). In some embodiments, the first liquid composition does not comprise a lipid (e.g. soybean oil). In some embodiments, the lipid is soybean oil.

In some embodiments, the said parenteral infusion composition of docetaxel, or a pharmaceutically acceptable salt thereof, does not comprise a surfactant. In some embodiments, the first liquid composition does not comprise a surfactant. In some embodiments, the second aqueous composition does not comprise a surfactant.

In some embodiments, the said parenteral infusion composition of docetaxel, or a pharmaceutically acceptable salt thereof, is prepared less than 24 hours prior to being infused or administered to patients. In some embodiments, the said parenteral infusion composition of docetaxel, or a pharmaceutically acceptable salt thereof, is prepared less than 8 hours prior to being infused or administered to patients. In some embodiments, the said parenteral infusion composition of docetaxel, or a pharmaceutically acceptable salt thereof, is prepared less than 6 hours prior to being infused or administered to patients. In some embodiments, the said parenteral infusion composition of docetaxel, or a pharmaceutically acceptable salt thereof, is prepared less than 4 hours prior to being infused or administered to patients. In some embodiments, the said parenteral infusion composition of docetaxel, or a pharmaceutically acceptable salt thereof, is prepared less than 1 hour prior to being infused or administered to patients.

In some embodiments, the concentration of docetaxel, or a pharmaceutically acceptable salt thereof, in the parenteral infusion composition is from about 0.1 mg/ml to about 0.8 mg/ml. In some embodiments, the concentration of docetaxel, or a pharmaceutically acceptable salt thereof, in the parenteral infusion composition is from about 0.1 mg/ml to about 0.5 mg/ml. In some embodiments, the concentration of docetaxel, or a pharmaceutically acceptable salt thereof, in the parenteral infusion composition is from about 0.2 mg/ml to about 0.4 mg/ml. In some embodiments, the concentration of docetaxel, or a pharmaceutically acceptable salt thereof, in the parenteral infusion composition is from about 0.25 mg/ml to about 0.35 mg/ml. In some embodiments, weight ratio of human serum albumin to docetaxel, or a pharmaceutically acceptable salt thereof, is from about 20:1 to about 200:1. In some embodiments, weight ratio of human serum albumin to docetaxel, or a pharmaceutically acceptable salt thereof, is about 20:1, about 30:1, about 40:1, about 50:1, about 80:1, about 90:1, about 95:1, about 100:1, or about 150:1.

In some embodiments, the first liquid composition comprises docetaxel, or a pharmaceutically acceptable salt thereof, ethanol, and other organic solvents (e.g., alcohols such as propylene glycol, or polyethylene glycol 300, etc.). In some embodiments, the first liquid composition comprises docetaxel, or a pharmaceutically acceptable salt thereof, ethanol, and propylene glycol. In some embodiments, the first liquid composition comprises docetaxel, or a pharmaceutically acceptable salt thereof, ethanol, and polyethylene glycol 300. In some embodiments, the first liquid composition further comprises an acid. In some embodiments, the first liquid composition comprises docetaxel, or a pharmaceutically acceptable salt thereof, ethanol, and an organic acid. The most preferred acid for use in accordance with the present invention is citric acid. In some embodiments, the first liquid composition comprises docetaxel, or a pharmaceutically acceptable salt thereof, ethanol, and citric acid. In some embodiments, the first liquid composition comprises docetaxel, or a pharmaceutically acceptable salt thereof, ethanol, propylene glycol, and citric acid. In some embodiments, the first liquid composition is an ethanol solution containing docetaxel, or a pharmaceutically acceptable salt thereof. In some embodiments, the first liquid composition is an ethanol solution containing docetaxel, or a pharmaceutically acceptable salt thereof, and citric acid. In some embodiments, the first liquid composition is an ethanol/propylene glycol solution containing docetaxel and citric acid. In some embodiments, the weight ratio of docetaxel, or a pharmaceutically acceptable salt thereof, and citric acid in the first liquid composition is from about 5000:1 to about 1:1. In some embodiments, the weight ratio of docetaxel, or a pharmaceutically acceptable salt thereof, and citric acid in the first liquid composition is from about 2000:1 to about 1:1. In some embodiments, the weight ratio of docetaxel, or a pharmaceutically acceptable salt thereof, and citric acid in the first liquid composition is from about 1000:1 to about 1:1. In some embodiments, the weight ratio of docetaxel, or a pharmaceutically acceptable salt thereof, and citric acid in the first liquid composition is from about 500:1 to about 1:1. In some embodiments, the weight ratio of docetaxel, or a pharmaceutically acceptable salt thereof, and citric acid in the first liquid composition is from about 5000:1 to about 5:1. In some embodiments, the weight ratio of docetaxel, or a pharmaceutically acceptable salt thereof, and citric acid in the first liquid composition is from about 2000:1 to about 5:1. In some embodiments, the weight ratio of docetaxel, or a pharmaceutically acceptable salt thereof, and citric acid in the first liquid composition is from about 1000:1 to about 5:1. In some embodiments, the weight ratio of docetaxel, or a pharmaceutically acceptable salt thereof, and citric acid in the first liquid composition is from about 500:1 to about 5:1. In some embodiments, the weight ratio of docetaxel, or a pharmaceutically acceptable salt thereof, and citric acid in the first liquid composition is from about 5000:1 to about 10:1. In some embodiments, the weight ratio of docetaxel, or a pharmaceutically acceptable salt thereof, and citric acid in the first liquid composition is from about 2000:1 to about 10:1. In some embodiments, the weight ratio of docetaxel, or a pharmaceutically acceptable salt thereof, and citric acid in the first liquid composition is from about 1000:1 to about 10:1. In some embodiments, the weight ratio of docetaxel, or a pharmaceutically acceptable salt thereof, and citric acid in the first liquid composition is from about 500:1 to about 10:1. In some embodiments, the weight ratio of docetaxel, or a pharmaceutically acceptable salt thereof, and citric acid in the first liquid composition is from about 2000:1 to about 100:1. In some embodiments, the weight ratio of docetaxel, or a pharmaceutically acceptable salt thereof, and citric acid in the first liquid composition is from about 1000:1 to about 100:1. In some embodiments, the weight ratio of docetaxel, or a pharmaceutically acceptable salt thereof, and citric acid in the first liquid composition is from about 500:1 to about 100:1. In some embodiments, the weight ratio of docetaxel, or a pharmaceutically acceptable salt thereof, and citric acid in the first liquid composition is from about 5:1 to about 20:1. In some embodiments, the weight ratio of docetaxel, or a pharmaceutically acceptable salt thereof, and citric acid in the first liquid composition is about 5:1, about 10:1, about 15:1, or about 20:1. In some embodiments, the concentration of citric acid in the first liquid composition is from about 0.005 mg/ml to about 10 mg/ml. In some embodiments, the concentration of citric acid in the first liquid composition is from about 0.01 mg/ml to about 2 mg/ml. In some embodiments, the concentration of citric acid in the first liquid composition is from about 0.01 mg/ml to about 1 mg/ml. In some embodiments, the concentration of citric acid in the first liquid composition is from about 0.02 mg/ml to about 0.5 mg/ml. In some embodiments, the concentration of citric acid in the first liquid composition is from about 0.02 mg/ml to about 0.2 mg/ml.

In some embodiments, the first liquid composition is prepared by dissolving docetaxel, or a pharmaceutically acceptable salt thereof, in an alcohol (e.g., ethanol). In some embodiments, the first liquid composition is prepared by dissolving docetaxel, or a pharmaceutically acceptable salt thereof, and citric acid in ethanol. In some embodiments, the first liquid composition is prepared by dissolving docetaxel, or a pharmaceutically acceptable salt thereof, in ethanol and propylene glycol. In some embodiments, the first liquid composition is prepared by dissolving docetaxel, or a pharmaceutically acceptable salt thereof, and citric acid in ethanol and propylene glycol.

In some embodiments, the second aqueous composition is prepared by adding a human serum albumin solution into a parenterally acceptable vehicle. In some embodiments, the second aqueous composition is prepared by adding a human serum albumin solution into a normal saline solution or dextrose solution. In some embodiments, the second aqueous composition is prepared by adding a solution of human serum albumin for infusion into a normal saline solution or dextrose solution. In some embodiments, the second aqueous composition is prepared by adding a 20% solution of human serum albumin for infusion (w/v) into a normal saline solution or dextrose solution. In some embodiments, the second aqueous composition is prepared by adding a 25% solution of human serum albumin for infusion (w/v) into a normal saline solution or dextrose solution. In some embodiments, the second aqueous composition is prepared by adding a 5% solution of human serum albumin for infusion (w/v) into a normal saline solution or dextrose solution. In some embodiments, the concentration of human serum albumin in the second aqueous composition is from about 0.1% to about 20% (w/v). In some embodiments, the concentration of human serum albumin in the second aqueous composition is from about 0.5% to about 10% (w/v). In some embodiments, the concentration of human serum albumin in the second aqueous composition is from 0.5% to 5% (w/v). In some embodiments, the concentration of human serum albumin in the second aqueous composition is from 1% to 3% (w/v). In some embodiments, the second aqueous composition comprises from about 1g to about 50g of human serum albumin. In some embodiments, the second aqueous composition comprises from about 2 g to about 20 g of human serum albumin.

The injection of the first liquid composition into the infusion bag or bottle containing the second aqueous composition is rapid. In some embodiments, the injection time is no more than 60 seconds. In some embodiments, the injection time is no more than 30 seconds. In some embodiments, the injection time is no more than 15 seconds. In some embodiments, the injection time is no more than 10 seconds. In some embodiments, the injection time is no more than 5 seconds.

In the process of injecting the first liquid composition into the infusion bag or bottle containing the second aqueous composition, mixing or agitation is not need. After the injection of the first liquid composition into the infusion bag or bottle is completed, the first liquid composition and second aqueous composition are mixed well (e.g., the bag containing the composition is gently inverted by hand) to obtain a transparent infusion solution free of precipitates. In some embodiments, the infusion bag or bottle containing the second aqueous composition is held still in the injection process. In some embodiments, the first liquid composition is injected underneath the liquid surface of the second aqueous composition in the injection process. In some embodiments, after the injection is completed, the infusion bag or bottle is gently inverted repeatedly to mix well of the first liquid composition and second aqueous composition. In some embodiments, after the injection is completed, the infusion bag or bottle is gently inverted until a transparent solution free of precipitates is obtained. In some embodiments, after the injection is completed, the infusion bag or bottle is gently inverted for from about 5 seconds to about 10 minutes. In some embodiments, after the injection is completed, the infusion bag or bottle is gently inverted for from about 10 seconds to about 5 minutes. In some embodiments, after the injection is completed, the infusion bag or bottle is gently inverted for from about 0.5 minute to about 3 minutes. In some embodiments, after the injection is completed, the first liquid composition and second aqueous composition are mixed well until a transparent infusion solution free of precipitates is obtained.

After the first liquid composition and second aqueous composition are mixed well followed the injection of the first liquid composition into the infusion bag or bottle comprising the second aqueous composition, a transparent infusion solution free of precipitates comprising human serum albumin, docetaxel, or a pharmaceutically acceptable salt thereof, and ethanol in a parenterally acceptable vehicle is obtained. In some embodiments, the transparent infusion solution further comprises citric acid. In some embodiments, a parenterally acceptable vehicle is a normal saline solution or dextrose solution. In some embodiments, the transparent infusion solution stays transparent free of precipitates for at least 1 hour. In some embodiments, the transparent infusion solution stays transparent free of precipitates for at least 2 hours. In some embodiments, the transparent infusion solution stays transparent free of precipitates for at least 3 hours. In some embodiments, the transparent infusion solution stays transparent free of precipitates for at least 4 hours. In some embodiments, the transparent infusion solution stays transparent free of precipitates for at least 6 hours. In some embodiments, the transparent infusion solution stays transparent free of precipitates for at least 8 hours. In some embodiments, the transparent infusion solution stays transparent free of precipitates for at least 1 hour, 2 hours, 3 hours, 4 hours, or 6 hours, when the transparent solution keeps at about 20-25 °C. In some embodiments, the transparent solution stays transparent free of precipitates for at least 2 hours, 4 hours, 6 hours, 8 hours, or 12 hours, when the transparent solution keeps at about 2-8 °C.

Also, provided herein is a kit for the preparation of a parenteral infusion solution comprising: a first container containing a composition comprising docetaxel, or a pharmaceutically acceptable salt thereof; and a second container containing a composition comprising human serum albumin. In some embodiments, the kit comprises a first container containing a liquid composition comprising docetaxel, or a pharmaceutically acceptable salt thereof, and a second container containing a liquid composition comprising human serum albumin.

In some embodiments, the first container does not comprise a lipid (e.g. soybean oil). In some embodiments, the lipid is soybean oil. In some embodiments, the first container does not comprise a surfactant. In some embodiments, the second container does not comprise a surfactant.

In some embodiments, the first container contains a liquid composition comprising docetaxel, or a pharmaceutically acceptable salt thereof, and ethanol. In some embodiments, the first container contains a liquid composition comprising docetaxel, or a pharmaceutically acceptable salt thereof, an acid, or a pharmaceutically acceptable salt thereof, and ethanol. In some embodiments, the first container contains a liquid composition comprising docetaxel, or a pharmaceutically acceptable salt thereof, an organic acid, and ethanol. In some embodiments, the first container contains a liquid composition comprising docetaxel, or a pharmaceutically acceptable salt thereof, ethanol, and other organic solvents (e.g., alcohols such as propylene glycol, polyethylene glycol 300, etc.). In some embodiments, the first container contains a liquid composition comprising docetaxel, or a pharmaceutically acceptable salt thereof, ethanol, and propylene glycol. In some embodiments, the first container contains a liquid composition comprising docetaxel, or a pharmaceutically acceptable salt thereof, ethanol, and polyethylene glycol 300. In some embodiments, the first container contains a liquid composition comprising docetaxel, or a pharmaceutically acceptable salt thereof, and citric acid. In some embodiments, the first container contains a liquid composition comprising docetaxel, or a pharmaceutically acceptable salt thereof, citric acid, and ethanol. In some embodiments, the first container contains a liquid composition comprising docetaxel, or a pharmaceutically acceptable salt thereof, citric acid, ethanol, and propylene glycol. In some embodiments, the first container contains an ethanol solution containing docetaxel, or a pharmaceutically acceptable salt thereof. In some embodiments, the first container contains an ethanol solution containing docetaxel, or a pharmaceutically acceptable salt thereof, and citric acid. In some embodiments, the first container contains an ethanol/propylene glycol solution containing docetaxel, or a pharmaceutically acceptable salt thereof, and citric acid. In some embodiments, the weight ratio of docetaxel, or a pharmaceutically acceptable salt thereof, and citric acid, in the first container is from about 5000:1 to about 1:1. In some embodiments, the weight ratio of docetaxel, or a pharmaceutically acceptable salt thereof, and citric acid in the first container is from about 2000:1 to about 1:1. In some embodiments, the weight ratio of docetaxel, or a pharmaceutically acceptable salt thereof, and citric acid in the first container is from about 1000:1 to about 1:1. In some embodiments, the weight ratio of docetaxel, or a pharmaceutically acceptable salt thereof, and citric acid in the first container is from about 500:1 to about 1:1. In some embodiments, the weight ratio of docetaxel, or a pharmaceutically acceptable salt thereof, and citric acid in the first container is from about 5000:1 to about 5:1. In some embodiments, the weight ratio of docetaxel, or a pharmaceutically acceptable salt thereof, and citric acid in the first container is from about 2000:1 to about 5:1. In some embodiments, the weight ratio of docetaxel, or a pharmaceutically acceptable salt thereof, and citric acid in the first container is from about 1000:1 to about 5:1. In some embodiments, the weight ratio of docetaxel, or a pharmaceutically acceptable salt thereof, and citric acid in the first container is from about 500:1 to about 5:1. In some embodiments, the weight ratio of docetaxel, or a pharmaceutically acceptable salt thereof, and citric acid in the first container is from about 5000:1 to about 10:1. In some embodiments, the weight ratio of docetaxel, or a pharmaceutically acceptable salt thereof, and citric acid in the first container is from about 2000:1 to about 10:1. In some embodiments, the weight ratio of docetaxel, or a pharmaceutically acceptable salt thereof, and citric acid in the first container is from about 1000:1 to about 10:1. In some embodiments, the weight ratio of docetaxel, or a pharmaceutically acceptable salt thereof, and citric acid in the first container is from about 500:1 to about 10:1. In some embodiments, the weight ratio of docetaxel, or a pharmaceutically acceptable salt thereof, and citric acid in the first container is from about 2000:1 to about 100:1. In some embodiments, the weight ratio of docetaxel, or a pharmaceutically acceptable salt thereof, and citric acid in the first container is from about 1000:1 to about 100:1. In some embodiments, the weight ratio of docetaxel, or a pharmaceutically acceptable salt thereof, and citric acid in the first container is from about 500:1 to about 100:1. In some embodiments, the concentration of citric acid in the first container is from about 0.005 mg/ml to about 10 mg/ml. In some embodiments, the concentration of citric acid in the first container is from about 0.01 mg/ml to about 2 mg/ml. In some embodiments, the concentration of citric acid in the first container is from about 0.01 mg/ml to about 1 mg/ml. In some embodiments, the concentration of citric acid in the first container is from about 0.02 mg/ml to about 0.5 mg/ml. In some embodiments, the concentration of citric acid in the first container is from about 0.02 mg/ml to about 0.2 mg/ml.

In some embodiments, the amount of docetaxel contained in the first container is from about 10 mg to about 300 mg of docetaxel. In some embodiments, the amount of docetaxel contained in the first container is from about 20 mg to about 200 mg of docetaxel. In some embodiments, the amount of docetaxel contained in the first container is about 20 mg, about 40 mg, about 60 mg, about 80 mg, about 100 mg, about 120 mg, or about 160 mg of docetaxel. In some embodiments, the amount of docetaxel contained in the first container is about 80 mg of docetaxel. In some embodiments, the amount of docetaxel contained in the first container is about 100 mg of docetaxel. In some embodiments, the amount of ethanol contained in the first container is from about 0.5 ml to about 50 ml of ethanol. In some embodiments, the amount of ethanol contained in the first container is from about 1 ml to about 20 ml of ethanol. In some embodiments, the amount of ethanol contained in the first container is from about 1 ml to about 10 ml of ethanol. In some embodiments, the amount of ethanol contained in the first container is from about 3 ml to about 6 ml of ethanol. In some embodiments, the concentration of docetaxel, or a pharmaceutically acceptable salt thereof, in the first container is from about 1 mg/ml to about 200 mg/ml. In some embodiments, the concentration of docetaxel, or a pharmaceutically acceptable salt thereof, in the first container is from about 5 mg/ml to about 100 mg/ml. In some embodiments, the concentration of docetaxel, or a pharmaceutically acceptable salt thereof, in the first container is from about 10 mg/ml to about 50 mg/ml. In some embodiments, the concentration of docetaxel in the first container is about 20 mg/ml. In some embodiments, concentration of docetaxel, or a pharmaceutically acceptable salt thereof, in the alcohol in the first container is from about 10 mg/ml to about 30 mg/ml. In some embodiments, concentration of docetaxel in the alcohol in the first container is about 10 mg/ml, about 20 mg/ml, or about 30 mg/ml.

In some embodiments, the first container contains a solid composition comprising docetaxel, or a pharmaceutically acceptable salt thereof,. In some embodiments, the first container contains a solid composition comprising docetaxel, or a pharmaceutically acceptable salt thereof, and an organic acid. In some embodiments, the first container contains a solid composition comprising docetaxel, or a pharmaceutically acceptable salt thereof, and citric acid.

In some embodiments, the second container contains a liquid composition comprising human serum albumin. In some embodiments, the second container contains a human serum albumin solution. In some embodiments, the second container contains a human serum albumin solution with the concentration of human serum albumin in the solution from about 1% to about 25% (w/v). In some embodiments, the second container contains a human serum albumin solution with the concentration of human serum albumin in the solution from about 5% to about 25% (w/v). In some embodiments, the second container contains a solution of human serum albumin for infusion. In some embodiments, the second container contains a 20% solution of human serum albumin for infusion (w/v). In some embodiments, the second container contains a 25% solution of human serum albumin for infusion (w/v). In some embodiments, the second container contains a 5% solution of human serum albumin for infusion (w/v). In some embodiments, the second container contains a liquid composition comprising from about 1 g to about 50 g of human serum albumin. In some embodiments, the second container contains a liquid composition comprising from about 1 g to about 30 g of human serum albumin. In some embodiments, the second container contains a liquid composition comprising from about 1 g to about 20 g of human serum albumin. In some embodiments, the second container contains a liquid composition comprising about 2 g, about 4 g, about 6 g, about 8 g, about 10 g, about 12 g, or about 16 g of human serum albumin.

In some embodiments, the kit further comprises instructions to add the composition comprising docetaxel, or a pharmaceutically acceptable salt thereof, to the composition comprising human serum albumin.

Also, provided herein a parenteral pharmaceutical formulation comprising two compositions that are mixed prior to being infused or administered to patients, that comprises: (a) a first liquid composition comprising a substantially water-insoluble pharmaceutically active agent, or a pharmaceutically acceptable salt thereof, and (b) a second aqueous composition comprising human serum albumin and a parenterally acceptable vehicle, and wherein the substantially water-insoluble pharmaceutically active agent, or a pharmaceutically acceptable salt thereof, is the API (active pharmaceutical ingredient) of the said parenteral pharmaceutical formulation.

In some embodiments, the said pharmaceutical formulation does not comprise a lipid (e.g. soybean oil). In some embodiments, the first liquid composition does not comprise a lipid (e.g. soybean oil). In some embodiments, the lipid is soybean oil.

In some embodiments, the said pharmaceutical formulation does not comprise a surfactant. In some embodiments, the first liquid composition does not comprise a surfactant. In some embodiments, the second aqueous composition does not comprise a surfactant.

### DESCRIPTION OF DRAWINGS

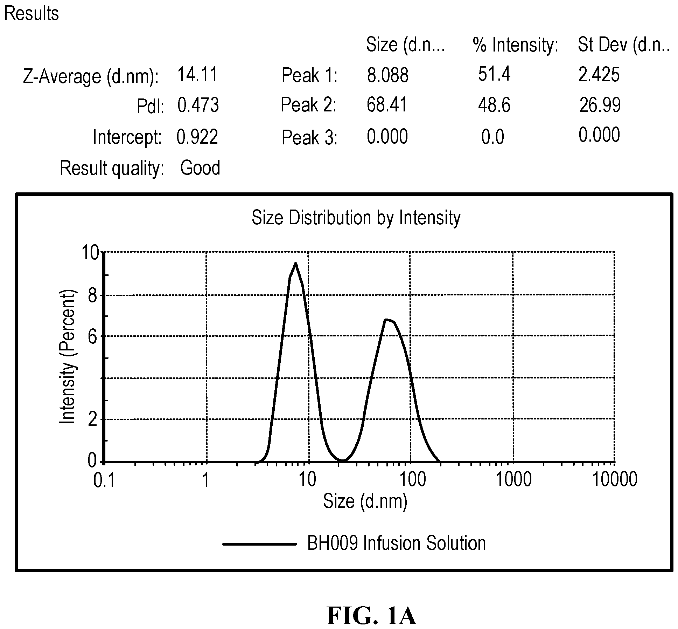

FIG 1A is a line plot showing DLS Test Results of Docetaxel Infusion Solution.

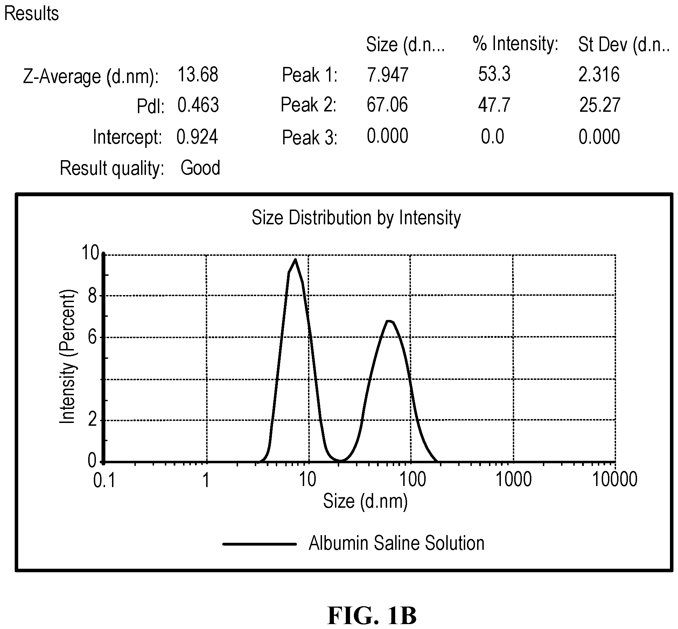

FIG 1B is a line plot showing DLS Test Results of Albumin Saline Solution.

### DETAILED DESCRIPTION

Provided herein is a pharmaceutical formulation of docetaxel, or a pharmaceutically acceptable salt thereof, comprising compositions that are mixed prior to being infused or administered to patients, that comprises: (a) a first liquid composition comprising docetaxel, or a pharmaceutically acceptable salt thereof, ethanol, and an organic acid, wherein the organic acid is not aspartic acid or glutamic acid, and (b) a second aqueous composition comprising human serum albumin and a parenterally acceptable vehicle, and wherein the pharmaceutical formulation does not contain Polysorbate 80. In some embodiments, first liquid composition comprising docetaxel, or a pharmaceutically acceptable salt thereof, citric acid, and ethanol. In some embodiments, the two compositions are mixed in less than 24 hours prior to being infused or administered to patients. In some embodiments, the mixing of the two compositions is done in an infusion bag or bottle. In some embodiments, the two compositions are mixed in less than 12 hours prior to being infused or administered to patients. In some embodiments, the two compositions are mixed in less than 8 hours prior to being infused or administered to patients. In some embodiments, the two compositions are mixed in less than 4 hours prior to being infused or administered to patients. In some embodiments, the two compositions are mixed in less than 1 hour prior to being infused or administered to patients. In some embodiments, the mixing is done in a infusion bag or bottle. In some embodiments, the second aqueous composition is contained in an infusion bag or bottle. In some embodiments, the first liquid composition is injected into an infusion bag or bottle which contains the second aqueous composition. In some embodiments, both the first liquid composition and the second aqueous composition are sterile solutions. In some embodiments, the first liquid composition is sterile solution. In some embodiments, the second aqueous composition is sterile solution. In some embodiments, the mixing of the first liquid composition and the second aqueous composition is done in a hospital or clinic prior to being infused or administered to patients.

In some embodiments, the said pharmaceutical formulation does not comprise a lipid (e.g. soybean oil). In some embodiments, the first liquid composition does not comprise a lipid (e.g. soybean oil). In some embodiments, the lipid is soybean oil.

In some embodiments, the said pharmaceutical formulation does not comprise a surfactant. In some embodiments, the first liquid composition does not comprise a surfactant. In some embodiments, the second aqueous composition does not comprise a surfactant.

In some embodiments, the mixing of the first liquid composition with the second aqueous composition includes two steps. In the first step, the first liquid composition is injected into the infusion bag or bottle containing the second aqueous composition. In the injection process, the infusion bag or bottle is held still and mixing or agitation is not need. In the second step, immediately after the injection, the infusion bag or bottle is inverted until a transparent infusion solution free of precipitates is obtained.

In the process of injecting the first liquid composition into the infusion bag or bottle containing the second aqueous composition, the infusion bag or bottle is held still and mixing or agitation is not need in the injection process. We surprisingly found that by simply inverting the infusion bag or bottle repeatedly (e.g. for 5-30 times) after the injection, a transparent infusion solution free of precipitates is obtained.

In some embodiments, the concentration of docetaxel, or a pharmaceutically acceptable salt thereof, in the formulation after mixing of the first liquid composition and the second aqueous composition is from about 0.05mg/ml to about 1mg/ml. In some embodiments, the concentration of docetaxel, or a pharmaceutically acceptable salt thereof, in the formulation after mixing of the first liquid composition and the second aqueous composition is from about 0.1mg/ml to about 0.5mg/ml. In some embodiments, the concentration of docetaxel, or a pharmaceutically acceptable salt thereof, in the formulation after mixing of the first liquid composition and the second aqueous composition is from about 0.15mg/ml to about 0.4mg/ml. In some embodiments, the concentration of docetaxel, or a pharmaceutically acceptable salt thereof, in the formulation after mixing of the first liquid composition and the second aqueous composition is from about 0.2mg/ml to about 0.3mg/ml.

In the present disclosure, the formulation obtained after mixing of the first liquid composition and the second aqueous composition is a clear infusion solution, not a nanoparticle suspension. In some embodiments, at least 30% docetaxel in the formulation (clear infusion solution) is Free (unbound) docetaxel. In some embodiments, at least 40% docetaxel in the formulation (clear infusion solution) is Free (unbound) docetaxel. In some embodiments, at least 50% docetaxel in the formulation (clear infusion solution) is Free (unbound) docetaxel. In some embodiments, at least 60% docetaxel in the formulation (clear infusion solution) is Free (unbound) docetaxel. In some embodiments, at least 70% docetaxel in the formulation (clear infusion solution) is Free (unbound) docetaxel. Free (unbound) docetaxel means the fraction of docetaxel in the infusion composition (solution) that is not bound to human serum albumin. Concentration of free (unbound) docetaxel in the parenteral infusion composition (solution) is measured by ultrafiltration through a 30-kDa membrane. The experimental details of free (unbound) docetaxel measurement are described in the example section.

In some embodiments, the pharmaceutical formulation of docetaxel, or a pharmaceutically acceptable salt thereof, with two compositions that are mixed prior to being infused or administered to patients, that comprises: (a) a first liquid composition comprising from about 20 mg to about 200 mg of docetaxel, or a pharmaceutically acceptable salt thereof, dissolved in an alcohol (e.g., ethanol), and (b) a second aqueous composition comprising from about 0.5 g to about 20 g of human serum albumin in a parenterally acceptable vehicle. In some embodiments, the pharmaceutical formulation of docetaxel, or a pharmaceutically acceptable salt thereof, with two compositions that are mixed prior to being infused or administered to patients, that comprises: (a) a first liquid composition comprising from about 20 mg to about 200 mg of docetaxel, or a pharmaceutically acceptable salt thereof, and citric acid dissolved in an alcohol (e.g., ethanol), and (b) a second aqueous composition comprising from about 0.5 g to about 20 g of human serum albumin in a parenterally acceptable vehicle. In some embodiments, the pharmaceutical formulation of docetaxel, or a pharmaceutically acceptable salt thereof, with two compositions that are mixed prior to being infused or administered to patients, that comprises: (a) a first liquid composition comprising from about 20 mg to about 200 mg of docetaxel, or a pharmaceutically acceptable salt thereof, dissolved in ethanol, and (b) a second aqueous composition comprising from about 1 g to about 10 g of human serum albumin in a parenterally acceptable vehicle. In some embodiments, the pharmaceutical formulation of docetaxel, or a pharmaceutically acceptable salt thereof, with two compositions that are mixed prior to being infused or administered to patients, that comprises: (a) a first liquid composition comprising from about 20 mg to about 200 mg of docetaxel, or a pharmaceutically acceptable salt thereof, and citric acid dissolved in ethanol, and (b) a second aqueous composition comprising from about 1 g to about 10 g of human serum albumin in a parenterally acceptable vehicle. In some embodiments, the pharmaceutical formulation does not comprise a lipid (e.g. soybean oil). In some embodiments, the pharmaceutical formulation does not comprise a surfactant.

In some embodiments, ethanol in the first liquid composition is dehydrated ethanol. In some embodiments, the first liquid composition comprises docetaxel, or a pharmaceutically acceptable salt thereof, ethanol, and other organic solvents (e.g., alcohols such as propylene glycol, polyethylene glycol 300, etc.). In some embodiments, the first liquid composition comprises docetaxel, or a pharmaceutically acceptable salt thereof, ethanol, and propylene glycol. In some embodiments, the first liquid composition comprises docetaxel, or a pharmaceutically acceptable salt thereof, ethanol, and polyethylene glycol 300. In some embodiments, the first liquid composition further comprises an acid. In some embodiments, the first liquid composition comprises docetaxel, or a pharmaceutically acceptable salt thereof, ethanol, and an organic acid. Preferably the organic acid is selected in the group consisting of citric acid, acetic acid, formic acid, ascorbic acid, benzoic acid, tartaric acid, lactic acid, maleic acid, and succinic acid, or a pharmaceutically acceptable salt thereof. The organic acid excludes aspartic acid and glutamic acid. The most preferred acid for use in accordance with the present invention is citric acid. In some embodiments, citric acid is anhydrous citric acid. In some embodiments, the first liquid composition comprises docetaxel, or a pharmaceutically acceptable salt thereof, ethanol, and citric acid. In some embodiments, the first liquid composition comprises docetaxel, or a pharmaceutically acceptable salt thereof, ethanol, propylene glycol, and citric acid. In some embodiments, the first liquid composition is an ethanol solution containing docetaxel, or a pharmaceutically acceptable salt thereof. In some embodiments, the first liquid composition is an ethanol solution containing docetaxel, or a pharmaceutically acceptable salt thereof, and citric acid. In some embodiments, the first liquid composition is an ethanol/propylene glycol solution containing docetaxel, or a pharmaceutically acceptable salt thereof, and citric acid. In some embodiments, the first liquid composition is a dehydrated ethanol solution containing docetaxel, or a pharmaceutically acceptable salt thereof, and citric acid. In some embodiments, the weight ratio of docetaxel, or a pharmaceutically acceptable salt thereof, and citric acid in the first liquid composition is from about 5000:1 to about 1:1. In some embodiments, the weight ratio of docetaxel, or a pharmaceutically acceptable salt thereof, and citric acid in the first liquid composition is from about 2000:1 to about 1:1. In some embodiments, the weight ratio of docetaxel, or a pharmaceutically acceptable salt thereof, and citric acid in the first liquid composition is from about 1000:1 to about 1:1. In some embodiments, the weight ratio of docetaxel, or a pharmaceutically acceptable salt thereof, and citric acid in the first liquid composition is from about 500:1 to about 1:1. In some embodiments, the weight ratio of docetaxel, or a pharmaceutically acceptable salt thereof, and citric acid in the first liquid composition is from about 5000:1 to about 5:1. In some embodiments, the weight ratio of docetaxel, or a pharmaceutically acceptable salt thereof, and citric acid in the first liquid composition is from about 2000:1 to about 5:1. In some embodiments, the weight ratio of docetaxel, or a pharmaceutically acceptable salt thereof, and citric acid in the first liquid composition is from about 1000:1 to about 5:1. In some embodiments, the weight ratio of docetaxel, or a pharmaceutically acceptable salt thereof, and citric acid in the first liquid composition is from about 500:1 to about 5:1. In some embodiments, the weight ratio of docetaxel, or a pharmaceutically acceptable salt thereof, and citric acid in the first liquid composition is from about 5000:1 to about 10:1. In some embodiments, the weight ratio of docetaxel, or a pharmaceutically acceptable salt thereof, and citric acid in the first liquid composition is from about 2000:1 to about 10:1. In some embodiments, the weight ratio of docetaxel, or a pharmaceutically acceptable salt thereof, and citric acid in the first liquid composition is from about 1000:1 to about 10:1. In some embodiments, the weight ratio of docetaxel, or a pharmaceutically acceptable salt thereof, and citric acid in the first liquid composition is from about 500:1 to about 10:1. In some embodiments, the weight ratio of docetaxel, or a pharmaceutically acceptable salt thereof, and citric acid in the first liquid composition is from about 2000:1 to about 100:1. In some embodiments, the weight ratio of docetaxel, or a pharmaceutically acceptable salt thereof, and citric acid in the first liquid composition is from about 1000:1 to about 100:1. In some embodiments, the weight ratio of docetaxel, or a pharmaceutically acceptable salt thereof, and citric acid in the first liquid composition is from about 500:1 to about 100:1. In some embodiments, the concentration of citric acid in the first liquid composition is from about 0.005 mg/ml to about 10 mg/ml. In some embodiments, the concentration of citric acid in the first liquid composition is from about 0.01 mg/ml to about 2 mg/ml. In some embodiments, the concentration of citric acid in the first liquid composition is from about 0.01 mg/ml to about 1 mg/ml. In some embodiments, the concentration of citric acid in the first liquid composition is from about 0.02 mg/ml to about 0.5 mg/ml. In some embodiments, the concentration of citric acid in the first liquid composition is from about 0.02 mg/ml to about 0.2 mg/ml. In some embodiments, the concentration of docetaxel, or a pharmaceutically acceptable salt thereof, in the first liquid composition is from about 1 mg/ml to about 200 mg/ml. In some embodiments, the concentration of docetaxel, or a pharmaceutically acceptable salt thereof, in the first liquid composition is from about 5 mg/ml to about 100 mg/ml. In some embodiments, the concentration of docetaxel, or a pharmaceutically acceptable salt thereof, in the first liquid composition is from about 10 mg/ml to about 50 mg/ml. In some embodiments, the concentration of docetaxel, or a pharmaceutically acceptable salt thereof, in the first liquid composition is about 20 mg/ml. In some embodiments, concentration of docetaxel, or a pharmaceutically acceptable salt thereof, in the first liquid composition is from about 10 mg/ml to about 30 mg/ml. In some embodiments, concentration of docetaxel, or a pharmaceutically acceptable salt thereof, in the first liquid composition is about 10 mg/ml, about 20 mg/ml, or about 30 mg/ml.

In some embodiments, the first liquid composition comprises docetaxel, or a pharmaceutically acceptable salt thereof, and a solvent including ethanol, wherein docetaxel, or a pharmaceutically acceptable salt thereof, is dissolved in the solvent. In some embodiments, the first liquid composition comprises docetaxel, or a pharmaceutically acceptable salt thereof, an acid, and a solvent including ethanol, wherein docetaxel, or a pharmaceutically acceptable salt thereof, and the acid are dissolved in the solvent. In some embodiments, the first liquid composition comprises docetaxel, or a pharmaceutically acceptable salt thereof, an organic acid, and a solvent including ethanol, wherein docetaxel, or a pharmaceutically acceptable salt thereof, and the organic acid are dissolved in the solvent. In some embodiments, the first liquid composition comprises docetaxel, or a pharmaceutically acceptable salt thereof, citric acid, and a solvent including ethanol, wherein docetaxel, or a pharmaceutically acceptable salt thereof, and citric acid are dissolved in the solvent. In some embodiments, the first liquid composition comprises docetaxel, or a pharmaceutically acceptable salt thereof, and ethanol, wherein docetaxel, or a pharmaceutically acceptable salt thereof, is dissolved in ethanol. In some embodiments, the first liquid composition comprises docetaxel, or a pharmaceutically acceptable salt thereof, citric acid, and ethanol, wherein docetaxel, or a pharmaceutically acceptable salt thereof, and citric acid are dissolved in ethanol. In some embodiments, the first liquid composition comprises docetaxel, or a pharmaceutically acceptable salt thereof, and dehydrated ethanol, wherein docetaxel, or a pharmaceutically acceptable salt thereof, is dissolved in dehydrated ethanol. In some embodiments, the first liquid composition comprises docetaxel, or a pharmaceutically acceptable salt thereof, citric acid, and dehydrated ethanol, wherein docetaxel, or a pharmaceutically acceptable salt thereof, and citric acid are dissolved in dehydrated ethanol. In some embodiments, the first liquid composition does not comprise a lipid (e.g. soybean oil) or a surfactant.

In some embodiments, the first liquid composition is prepared by dissolving docetaxel, or a pharmaceutically acceptable salt thereof, in ethanol. In some embodiments, the first liquid composition is prepared by dissolving docetaxel, or a pharmaceutically acceptable salt thereof, and citric acid in ethanol. In some embodiments, the first liquid composition is prepared by dissolving docetaxel, or a pharmaceutically acceptable salt thereof, in ethanol and propylene glycol. In some embodiments, the first liquid composition is prepared by dissolving docetaxel, or a pharmaceutically acceptable salt thereof, and citric acid in ethanol and propylene glycol.

In some embodiments, the second aqueous composition is prepared by adding a human serum albumin solution into a parenterally acceptable vehicle. In some embodiments, the second aqueous composition is prepared by adding a human serum albumin solution into an infusion bag or bottle which contains a parenterally acceptable vehicle. In some embodiments, the second aqueous composition is prepared by adding a human serum albumin solution into a normal saline solution or dextrose solution. In some embodiments, the second aqueous composition is prepared by adding a human serum albumin solution into an infusion bag or bottle which contains a normal saline solution or dextrose solution. In some embodiments, the second aqueous composition is prepared by adding a solution of human serum albumin for infusion into a parenterally acceptable vehicle. In some embodiments, the second aqueous composition is prepared by adding a solution of human serum albumin for infusion into a normal saline solution or dextrose solution. In some embodiments, the second aqueous composition is prepared by adding a 20% solution of human serum albumin for infusion (w/v) into a normal saline solution or dextrose solution. In some embodiments, the second aqueous composition is prepared by adding a 25% solution of human serum albumin for infusion (w/v) into a normal saline solution or dextrose solution. In some embodiments, the second aqueous composition is prepared by adding a 5% solution of human serum albumin for infusion (w/v) into a normal saline solution or dextrose solution. In some embodiments, the second aqueous composition is prepared by adding a solution of human serum albumin for infusion into an infusion bag or bottle which contains a normal saline solution or dextrose solution. In some embodiments, the second aqueous composition is prepared by adding a solution of human serum albumin for infusion into an infusion bag or bottle which contains a normal saline solution or dextrose solution. In some embodiments, the second aqueous composition is prepared by adding a 20% solution of human serum albumin for infusion (w/v) into an infusion bag or bottle which contains a normal saline solution or dextrose solution. In some embodiments, the second aqueous composition is prepared by adding a 25% solution of human serum albumin for infusion (w/v) into an infusion bag or bottle which contains a normal saline solution or dextrose solution. In some embodiments, the second aqueous composition is prepared by adding a 5% solution of human serum albumin for infusion (w/v) into an infusion bag or bottle which contains a normal saline solution or dextrose solution. In some embodiments, the concentration of human serum albumin in the second aqueous composition is from about 0.1% to about 20% (w/v). In some embodiments, the concentration of human serum albumin in the second aqueous composition is from about 0.5% to about 15% (w/v). In some embodiments, the concentration of human serum albumin in the second aqueous composition is from about 0.5% to about 10% (w/v). In some embodiments, the concentration of human serum albumin in the second aqueous composition is from 0.5% to 5% (w/v). In some embodiments, the concentration of human serum albumin in the second aqueous composition is from about 1% to about 10% (w/v). In some embodiments, the concentration of human serum albumin in the second aqueous composition is from 1% to 3% (w/v). In some embodiments, the second aqueous composition comprises from about 1 g to about 50 g of human serum albumin. In some embodiments, the second aqueous composition comprises from about 1g to about 20 g of human serum albumin. In some embodiments, the second aqueous composition comprises from about 2g to about 10 g of human serum albumin. In some embodiments, the volume of the second aqueous composition is from about 100 ml to about 1L. In some embodiments, the volume of the second aqueous composition is from about 250 ml to about 1L. In some embodiments, the volume of the second aqueous composition is from about 250 ml to about 500ml.

As used herein the term "docetaxel" refers to a compound that has the CAS No. 114977-28-5 and the following chemical structure: 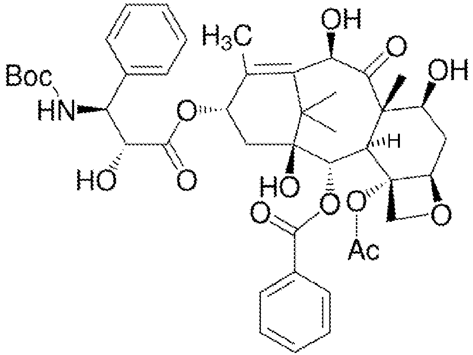 or a pharmaceutically acceptable salt thereof.

Docetaxel is a white to almost-white powder. It is highly lipophilic and practically insoluble in water.

Further, docetaxel is a microtubule inhibitor indicated for breast cancer, non-small cell lung cancer, hormone refractory prostate cancer, gastric adenocarcinoma, and squamous cell carcinoma of the head and neck cancer.

As used herein, the term "pharmaceutically acceptable salts" refers to salts that retain the desired biological activity of the subject compound and exhibit minimal undesired toxicological effects. These pharmaceutically acceptable salts may be prepared in situ during the final isolation and purification of the compound, or by separately reacting the purified compound in its free acid or free base form with a suitable base or acid, respectively. In some embodiments, pharmaceutically acceptable salts may be preferred over the respective free base or free acid because such salts impart greater stability or solubility to the molecule thereby facilitating formulation into a dosage form. Basic compounds are generally capable of forming pharmaceutically acceptable acid addition salts by treatment with a suitable acid. Suitable acids include pharmaceutically acceptable inorganic acids and pharmaceutically acceptable organic acids. Representative pharmaceutically acceptable acid addition salts include hydrochloride, hydrobromide, nitrate, methylnitrate, sulfate, bisulfate, sulfamate, phosphate, acetate, hydroxyacetate, phenylacetate, propionate, butyrate, isobutyrate, valerate, maleate, hydroxymaleate, acrylate, fumarate, malate, tartrate, citrate, salicylate, p-aminosalicyclate, glycollate, lactate, heptanoate, phthalate, oxalate, succinate, benzoate, o-acetoxybenzoate, chlorobenzoate, methylbenzoate, dinitrobenzoate, hydroxybenzoate, methoxybenzoate, mandelate, tannate, formate, stearate, ascorbate, palmitate, oleate, pyruvate, pamoate, malonate, laurate, glutarate, glutamate, estolate, methanesulfonate (mesylate), ethanesulfonate (esylate), 2-hydroxyethanesulfonate, benzenesulfonate (besylate), p-aminobenzenesulfonate, p-toluenesulfonate (tosylate),napthalene-2-sulfonate, ethanedisulfonate, hydrogen bisulfide, bitartrate, gluconate, glucuronate, parabromophenylsulfonate, carbonate, pyrosulfate, sulfite, bisulfite, monohydrogen phosphate, dihydrogen phosphate, metaphosphate, pyrophosphate, chloride, bromide, iodide, decanoate, caprylate, caprate, propiolate, suberate, sebacate, butyne-1,4-dioate, hexyne-1,6-dioate, terephthalate, sulfonate, xylenesulfonate, phenylpropionate, phenylbutyrate, β-hydroxybutyrate, glycolate, propanesulfonate, naphthalene-1-sulfonate, naphthalene-2-sulfonate and 2,5-dihydroxybenzoate. Suitable bases include pharmaceutically acceptable inorganic bases and pharmaceutically acceptable organic bases. Representative pharmaceutically acceptable base addition salts include hydroxide of alkali metals including sodium, potassium, and lithium; hydroxides of alkaline earth metals such as calcium and magnesium; hydroxides of other metals, such as aluminum and zinc; ammonia, organic amines such as unsubstituted or hydroxyl-substituted mono-, di-, or tri-alkylamines, dicyclohexylamine; tributyl amine; pyridine; N-methyl, N-ethylamine; diethylamine; triethylamine; mono-, bis-, or tris-(2-OH-(C₁-C₆)-alkylamine), such as N,N-dimethyl-N-(2-hydroxyethyl)amine or tri-(2-hydroxyethyl)amine; N-methyl-D-glucamine; morpholine; thiomorpholine; piperidine; pyrrolidine; and amino acids such as arginine, lysine, and the like.

In some embodiments, the docetaxel, or a pharmaceutically acceptable salt thereof, can be a docetaxel with 1, 2, or 3 equivalents of the water solvate. In some embodiments, the docetaxel, or a pharmaceutically acceptable salt thereof, can be a docetaxel with three equivalents of the water solvate. In some embodiments, docetaxel is the docetaxel trihydrate, or a pharmaceutically acceptable salt thereof. In some embodiments, docetaxel is the docetaxel monohydrate, or a pharmaceutically acceptable salt thereof. In some embodiments, docetaxel is the docetaxel anhydrous, or a pharmaceutically acceptable salt thereof. In some embodiments, the docetaxel can be a docetaxel with one equivalent of the acetone solvate. In some embodiments, the docetaxel can be any one of docetaxel solvates disclosed, for example, in WO2010091650 or US2012007167, or a pharmaceutically acceptable salt thereof.

In some embodiments, docetaxel, or a pharmaceutically acceptable salt thereof, is crystalline. In some embodiments, docetaxel, or a pharmaceutically acceptable salt thereof, is any one of the crystalline forms disclosed, for example, in WO2012115402, US8410294, US20100197944, US20100099897, US8357811, US20100160653, or US20070142457.

In some embodiments, docetaxel, or a pharmaceutically acceptable salt thereof, in amorphous. In some embodiments. docetaxel is any one of the amorphous forms disclosed, for example, in WO2008102374, or a pharmaceutically acceptable salt thereof.

As used herein, the term "human serum albumin" refers to native and recombinant human serum albumin. In some embodiments, the human serum albumin is a native human serum albumin. In some embodiments, the human serum albumin is a recombinant human serum albumin.

Suitable human serum albumin solution include, but are not limited to, commercially available solutions of human serum albumin for infusion. The commercially available solutions of human serum albumin for infusion comprise pharmaceutically acceptable excipient(s) such as sodium N-acetyltryptophan, sodium caprylate, sodium chloride, sodium bicarbonate, sodium hydroxide, or acetic acid, and the like or mixtures thereof. In some embodiments, human serum albumin solution can be prepared by diluting commercially available solutions of human serum albumin for infusion with a parenterally acceptable vehicle.

Alternatively, human serum albumin solution can be prepared by mixing human serum albumin powder in water along with other pharmaceutically acceptable excipient(s) as available in the commercially available albumin products.

In some embodiments, the human serum albumin solution is a commercially available solution of human serum albumin USP for infusion. In some embodiments, the human serum albumin solution for infusion.is a commercially available solution of human serum albumin USP for infusion. In some embodiments, the human serum albumin solution comprises a commercially available solution of human serum albumin USP for infusion. In some embodiments, a commercially available solution of human serum albumin USP for infusion is used as the source of the human serum albumin solution. In some embodiments, the solution of human serum albumin for infusion is 5% solution of human serum albumin USP (w/v). In some embodiments, the solution of human serum albumin for infusion is 20% solution of human serum albumin USP (w/v). In some embodiments, the solution of human serum albumin for infusion is 25% solution of human serum albumin USP (w/v). In some embodiments, the human serum albumin solution is an aqueous solution prepared by diluting a commercially available solution of human serum albumin for infusion.

The term "parenteral" refers to routes selected from subcutaneous (SC), intravenous (IV), intramuscular (IM), intrademal (ID), intraperitoneal (IP) and the like.

Also, provided herein is a transparent parenteral infusion composition of docetaxel, or a pharmaceutically acceptable salt thereof, free of precipitates comprising human serum albumin and said docetaxel, or a pharmaceutically acceptable salt thereof, in a concentration of docetaxel from about 0.05 mg/ml to about 1 mg/ml in a parenterally acceptable vehicle, wherein the said parenteral infusion solution is obtained by injecting a first liquid composition comprising docetaxel, or a pharmaceutically acceptable salt thereof, and ethanol into an infusion bag or bottle containing a second aqueous composition comprising human serum albumin in a parenterally acceptable vehicle, wherein mixing or agitation is not needed in the injection process, and wherein the said infusion solution does not contain Polysorbate 80. In some embodiments, a parenterally acceptable vehicle is a normal saline solution or dextrose solution.

In some embodiments, the said parenteral infusion composition of docetaxel, or a pharmaceutically acceptable salt thereof, does not comprise a lipid (e.g. soybean oil). In some embodiments, the first liquid composition does not comprise a lipid (e.g. soybean oil). In some embodiments, the lipid is soybean oil.

In some embodiments, the said parenteral infusion composition of docetaxel, or a pharmaceutically acceptable salt thereof, does not comprise a surfactant. In some embodiments, the first liquid composition does not comprise a surfactant. In some embodiments, the second aqueous composition does not comprise a surfactant.

In the present disclosure, the parenteral infusion composition of docetaxel, or a pharmaceutically acceptable salt thereof, is a clear solution, not a nanoparticle suspension. Measured by Dynamic Light Scattering (DLS), the parenteral infusion composition (solution) has almost identical profile in DLS compared to the related human serum albumin saline solution. The detailed DLS data are described in the example section. In some embodiments, at least 30% docetaxel in the parenteral infusion composition (solution) is Free (unbound) docetaxel. In some embodiments, at least 40% docetaxel in the parenteral infusion composition (solution) is Free (unbound) docetaxel. In some embodiments, at least 50% docetaxel in the parenteral infusion composition (solution) is Free (unbound) docetaxel. In some embodiments, at least 60% docetaxel in the parenteral infusion composition (solution) is Free (unbound) docetaxel. In some embodiments, at least 70% docetaxel in the parenteral infusion composition (solution) is Free (unbound) docetaxel. Free (unbound) docetaxel means the fraction of docetaxel in the parenteral infusion composition (solution) that is not bound to human serum albumin. Concentration of free (unbound) docetaxel in the parenteral infusion composition (solution) is measured by ultrafiltration through a 30-kDa membrane. The experimental details of free (unbound) docetaxel measurement are described in the example section.

In some embodiments, the amount of ethanol in the parenteral infusion composition of docetaxel, or a pharmaceutically acceptable salt thereof, is no more than 10% (v/v). In some embodiments, the amount of ethanol in the parenteral infusion composition of docetaxel, or a pharmaceutically acceptable salt thereof, is no more than 5% (v/v). In some embodiments, the amount of ethanol in the parenteral infusion composition of docetaxel, or a pharmaceutically acceptable salt thereof, is no more than 2% (v/v). In some embodiments, the amount of ethanol in the parenteral infusion composition of docetaxel, or a pharmaceutically acceptable salt thereof, is no more than 1.75% (v/v). In some embodiments, the amount of ethanol in the parenteral infusion composition of docetaxel, or a pharmaceutically acceptable salt thereof, is no more than 1.6% (v/v). In some embodiments, the amount of ethanol in the parenteral infusion composition of docetaxel, or a pharmaceutically acceptable salt thereof, is no more than 1.5% (v/v). In some embodiments, the amount of ethanol in the parenteral infusion composition of docetaxel, or a pharmaceutically acceptable salt thereof, is no more than 1.4% (v/v). In some embodiments, the amount of ethanol in the parenteral infusion composition of docetaxel, or a pharmaceutically acceptable salt thereof, is no more than 30 ml. In some embodiments, the amount of ethanol in the parenteral infusion composition of docetaxel, or a pharmaceutically acceptable salt thereof, is no more than 20 ml. In some embodiments, the amount of ethanol in the parenteral infusion composition of docetaxel, or a pharmaceutically acceptable salt thereof, is no more than 15 ml. In some embodiments, the amount of ethanol in the parenteral infusion composition of docetaxel, or a pharmaceutically acceptable salt thereof, is no more than 10 ml. In some embodiments, the amount of ethanol in the parenteral infusion composition of docetaxel, or a pharmaceutically acceptable salt thereof, is no more than 8 ml. In some embodiments, the amount of ethanol in the parenteral infusion composition of docetaxel, or a pharmaceutically acceptable salt thereof, is no more than 5 ml.

In some embodiments, the said parenteral infusion composition of docetaxel, or a pharmaceutically acceptable salt thereof, is prepared less than about 24 hours prior to being infused or administered to patients. In some embodiments, the said parenteral infusion composition of docetaxel, or a pharmaceutically acceptable salt thereof, is prepared less than about 12 hours prior to being infused or administered to patients. In some embodiments, the said parenteral infusion composition of docetaxel, or a pharmaceutically acceptable salt thereof, is prepared less than about 8 hours prior to being infused or administered to patients. In some embodiments, the said parenteral infusion composition of docetaxel, or a pharmaceutically acceptable salt thereof, is prepared less than about 6 hours prior to being infused or administered to patients. In some embodiments, the said parenteral infusion composition of docetaxel, or a pharmaceutically acceptable salt thereof, is prepared less than about 4 hours prior to being infused or administered to patients. In some embodiments, the said parenteral infusion composition of docetaxel, or a pharmaceutically acceptable salt thereof, is prepared less than about 1 hour prior to being infused or administered to patients.

In some embodiments, the pH value of the parenteral infusion composition is from about 4 to about 9.5. In some embodiments, the pH value of the parenteral infusion composition is from about 5 to about 9. In some embodiments, the pH value of the parenteral infusion composition is from about 6 to about 8. In some embodiments, the pH value of the parenteral infusion composition is from about 6.5 to about 7.5. In some embodiments, the pH value of the parenteral infusion composition is from about 4 to about 9. In some embodiments, the pH value of the parenteral infusion composition is from about 5 to about 8.5. In some embodiments, the pH value of the parenteral infusion composition is from about 6 to about 7.5.

In some embodiments, 1ml of the parenteral infusion composition comprises no more than 50 particles that are greater than 10 µm in size. In some embodiments, 1ml of the parenteral infusion composition comprises no more than 100 particles that are greater than 10 µm in size. In some embodiments, 1ml of the parenteral infusion composition comprises no more than 150 particles that are greater than 10 µm in size. In some embodiments, 1ml of the parenteral infusion composition comprises no more than 200 particles that are greater than 10 µm in size.
In some embodiments, 1ml of the parenteral infusion composition comprises no more than 5 particles that are greater than 25 µm in size. In some embodiments, 1ml of the parenteral infusion composition comprises no more than 10 particles that are greater than 25 µm in size. In some embodiments, 1ml of the parenteral infusion composition comprises no more than 5 particles that are greater than 15 µm in size. In some embodiments, 1ml of the parenteral infusion composition comprises no more than 20 particles that are greater than 25 µm in size.

In some embodiments, the concentration of docetaxel, or a pharmaceutically acceptable salt thereof, in the parenteral infusion composition is from about 0.1 mg/ml to about 0.8 mg/ml. In some embodiments, the concentration of docetaxel, or a pharmaceutically acceptable salt thereof, in the parenteral infusion composition is from about 0.1 mg/ml to about 0.5 mg/ml. In some embodiments, the concentration of docetaxel, or a pharmaceutically acceptable salt thereof, in the parenteral infusion composition is from about 0.15 mg/ml to about 0.4 mg/ml. In some embodiments, the concentration of docetaxel, or a pharmaceutically acceptable salt thereof, in the parenteral infusion composition is from about 0.2 mg/ml to about 0.35 mg/ml. In some embodiments, the concentration of docetaxel, or a pharmaceutically acceptable salt thereof, in the parenteral infusion composition is from about 0.1 mg/ml to about 0.3 mg/ml. In some embodiments, the concentration of docetaxel, or a pharmaceutically acceptable salt thereof, in the parenteral infusion composition is from about 0.2 mg/ml to about 0.3 mg/ml. In some embodiments, the concentration of docetaxel, or a pharmaceutically acceptable salt thereof, in the parenteral infusion composition is about 0.25 mg/ml. In some embodiments, weight ratio of human serum albumin to docetaxel, or a pharmaceutically acceptable salt thereof, is from about 20:1 to about 200: 1. In some embodiments, weight ratio of human serum albumin to docetaxel, or a pharmaceutically acceptable salt thereof, is from about 20:1 to about 100:1. In some embodiments, weight ratio of human serum albumin to docetaxel, or a pharmaceutically acceptable salt thereof, is about 20:1, about 30:1, about 40:1, about 50:1, about 60, about 70, about 80:1, about 90:1, about 95:1, about 100:1, or about 150:1. In some embodiments, the concentration of human serum albumin in the parenteral infusion composition is from about 0.1% to about 20% (w/v). In some embodiments, the concentration of human serum albumin in the parenteral infusion composition is from about 0.5% to about 20% (w/v). In some embodiments, the concentration of human serum albumin in the parenteral infusion composition is from about 0.5% to about 10% (w/v). In some embodiments, the concentration of human serum albumin in the parenteral infusion composition is from about 0.5% to about 5% (w/v). In some embodiments, the concentration of human serum albumin in the parenteral infusion composition is from about 1% to about 10% (w/v). In some embodiments, the concentration of human serum albumin in the parenteral infusion composition is from about 1% to about 5% (w/v). In some embodiments, the concentration of human serum albumin in the parenteral infusion composition is from about 0.5% to about 3% (w/v). In some embodiments, the concentration of human serum albumin in the parenteral infusion composition is from about 1% to about 3% (w/v).

In some embodiments, ethanol in the first liquid composition is dehydrated ethanol. In some embodiments, the first liquid composition comprises docetaxel, or a pharmaceutically acceptable salt thereof, ethanol, and other organic solvents (e.g., alcohol such as propylene glycol, or polyethylene glycol 300, etc.). In some embodiments, the first liquid composition comprises docetaxel, or a pharmaceutically acceptable salt thereof, ethanol, and propylene glycol. In some embodiments, the first liquid composition comprises docetaxel, or a pharmaceutically acceptable salt thereof, ethanol, and polyethylene glycol 300. In some embodiments, the first liquid composition further comprises an acid. In some embodiments, the first liquid composition comprises docetaxel, or a pharmaceutically acceptable salt thereof, ethanol, and an organic acid. The most preferred acid for use in accordance with the present invention is citric acid. In some embodiments, citric acid is anhydrous citric acid. In some embodiments, ethanol is dehydrated ethanol. In some embodiments, the first liquid composition comprises docetaxel, or a pharmaceutically acceptable salt thereof, ethanol, and citric acid. In some embodiments, the first liquid composition comprises docetaxel, or a pharmaceutically acceptable salt thereof, ethanol, propylene glycol, and citric acid. In some embodiments, the first liquid composition is an ethanol solution containing docetaxel, or a pharmaceutically acceptable salt thereof. In some embodiments, the first liquid composition is an ethanol solution containing docetaxel, or a pharmaceutically acceptable salt thereof, and citric acid. In some embodiments, the first liquid composition is an ethanol/propylene glycol solution containing docetaxel, or a pharmaceutically acceptable salt thereof, and citric acid. In some embodiments, the weight ratio of docetaxel, or a pharmaceutically acceptable salt thereof, and citric acid, in the first liquid composition is from about 5000:1 to about 1:1. In some embodiments, the weight ratio of docetaxel, or a pharmaceutically acceptable salt thereof, and citric acid in the first liquid composition is from about 2000:1 to about 1:1. In some embodiments, the weight ratio of docetaxel, or a pharmaceutically acceptable salt thereof, and citric acid in the first liquid composition is from about 1000:1 to about 1:1. In some embodiments, the weight ratio of docetaxel, or a pharmaceutically acceptable salt thereof, and citric acid in the first liquid composition is from about 500:1 to about 1:1. In some embodiments, the weight ratio of docetaxel, or a pharmaceutically acceptable salt thereof, and citric acid in the first liquid composition is from about 5000:1 to about 5:1. In some embodiments, the weight ratio of docetaxel, or a pharmaceutically acceptable salt thereof, and citric acid in the first liquid composition is from about 2000:1 to about 5:1. In some embodiments, the weight ratio of docetaxel, or a pharmaceutically acceptable salt thereof, and citric acid in the first liquid composition is from about 1000:1 to about 5:1. In some embodiments, the weight ratio of docetaxel, or a pharmaceutically acceptable salt thereof, and citric acid in the first liquid composition is from about 500:1 to about 5:1. In some embodiments, the weight ratio of docetaxel, or a pharmaceutically acceptable salt thereof, and citric acid in the first liquid composition is from about 5000:1 to about 10:1. In some embodiments, the weight ratio of docetaxel, or a pharmaceutically acceptable salt thereof, and citric acid in the first liquid composition is from about 2000:1 to about 10:1. In some embodiments, the weight ratio of docetaxel, or a pharmaceutically acceptable salt thereof, and citric acid in the first liquid composition is from about 1000:1 to about 10:1. In some embodiments, the weight ratio of docetaxel, or a pharmaceutically acceptable salt thereof, and citric acid in the first liquid composition is from about 500:1 to about 10:1. In some embodiments, the weight ratio of docetaxel, or a pharmaceutically acceptable salt thereof, and citric acid in the first liquid composition is from about 2000:1 to about 100:1. In some embodiments, the weight ratio of docetaxel, or a pharmaceutically acceptable salt thereof, and citric acid in the first liquid composition is from about 1000:1 to about 100:1. In some embodiments, the weight ratio of docetaxel, or a pharmaceutically acceptable salt thereof, and citric acid in the first liquid composition is from about 500:1 to about 100:1. In some embodiments, the weight ratio of docetaxel, or a pharmaceutically acceptable salt thereof, and citric acid in the first liquid composition is from about 5:1 to about 20:1. In some embodiments, the weight ratio of docetaxel, or a pharmaceutically acceptable salt thereof, and citric acid in the first liquid composition is about 5:1, about 10:1, about 15:1, or about 20:1. In some embodiments, the concentration of citric acid in the first liquid composition is from about 0.005 mg/ml to about 10 mg/ml. In some embodiments, the concentration of citric acid in the first liquid composition is from about 0.01 mg/ml to about 2 mg/ml. In some embodiments, the concentration of citric acid in the first liquid composition is from about 0.01 mg/ml to about 1 mg/ml. In some embodiments, the concentration of citric acid in the first liquid composition is from about 0.02 mg/ml to about 0.5 mg/ml. In some embodiments, the concentration of citric acid in the first liquid composition is from about 0.02 mg/ml to about 0.2 mg/ml. In some embodiments, the concentration of docetaxel, or a pharmaceutically acceptable salt thereof, in the first liquid composition is from about 1 mg/ml to about 200 mg/ml. In some embodiments, the concentration of docetaxel, or a pharmaceutically acceptable salt thereof, in the first liquid composition is from about 5 mg/ml to about 100 mg/ml. In some embodiments, the concentration of docetaxel, or a pharmaceutically acceptable salt thereof, in the first liquid composition is from about 10 mg/ml to about 50 mg/ml. In some embodiments, the concentration of docetaxel, or a pharmaceutically acceptable salt thereof, in the first liquid composition is about 20 mg/ml. In some embodiments, concentration of docetaxel, or a pharmaceutically acceptable salt thereof, in the first liquid composition is from about 10 mg/ml to about 30 mg/ml. In some embodiments, concentration of docetaxel, or a pharmaceutically acceptable salt thereof, in the first liquid composition is about 10 mg/ml, about 20 mg/ml, or about 30 mg/ml.

In some embodiments, the first liquid composition is prepared by dissolving docetaxel, or a pharmaceutically acceptable salt thereof, in ethanol. In some embodiments, the first liquid composition is prepared by dissolving docetaxel, or a pharmaceutically acceptable salt thereof, and citric acid in ethanol. In some embodiments, the first liquid composition is prepared by dissolving docetaxel, or a pharmaceutically acceptable salt thereof, in ethanol and propylene glycol. In some embodiments, the first liquid composition is prepared by dissolving docetaxel, or a pharmaceutically acceptable salt thereof, and citric acid in ethanol and propylene glycol.

In some embodiments, the second aqueous composition is prepared by adding a human serum albumin solution into a parenterally acceptable vehicle. In some embodiments, the second aqueous composition is prepared by adding a human serum albumin solution into a normal saline solution or dextrose solution. In some embodiments, the second aqueous composition is prepared by adding a solution of human serum albumin for infusion into a parenterally acceptable vehicle. In some embodiments, the second aqueous composition is prepared by adding a solution of human serum albumin for infusion into a normal saline solution or dextrose solution. In some embodiments, the second aqueous composition is prepared by adding a 20% solution of human serum albumin for infusion (w/v) into a normal saline solution or dextrose solution. In some embodiments, the second aqueous composition is prepared by adding a 25% solution of human serum albumin for infusion (w/v) into a normal saline solution or dextrose solution. In some embodiments, the second aqueous composition is prepared by adding a 5% solution of human serum albumin for infusion (w/v) into a normal saline solution or dextrose solution. In some embodiments, the concentration of human serum albumin in the second aqueous composition is from about 0.1% to about 20% (w/v). In some embodiments, the concentration of human serum albumin in the second aqueous composition is from about 0.5% to about 10% (w/v). In some embodiments, the concentration of human serum albumin in the second aqueous composition is from 0.5% to 5% (w/v). In some embodiments, the concentration of human serum albumin in the second aqueous composition is from 1% to 3% (w/v). In some embodiments, the second aqueous composition comprises from about 1 g to about 50 g of human serum albumin. In some embodiments, the second aqueous composition comprises from about 1 g to about 20 g of human serum albumin. In some embodiments, the second aqueous composition comprises from about 2 g to about 10 g of human serum albumin.

The injection of the first liquid composition into the infusion bag or bottle containing the second aqueous composition is rapid. In some embodiments, the injection time is no more than 60 seconds. In some embodiments, the injection time is no more than 30 seconds. In some embodiments, the injection time is no more than 15 seconds. In some embodiments, the injection time is no more than 10 seconds. In some embodiments, the injection time is no more than 5 seconds. In some embodiments, the injection time is no more than 3 seconds. In some embodiments, the injection of the first liquid composition into the infusion bag or bottle containing the second aqueous composition is done in the temperature from about 15 °C to about 30 °C. In some embodiments, the injection of the first liquid composition into the infusion bag or bottle containing the second aqueous composition is done in the temperature from about 15 °C to about 25 °C. In some embodiments, the injection of the first liquid composition into the infusion bag or bottle containing the second aqueous composition is done in the temperature from about 18 °C to about 25 °C. In some embodiments, the injection of the first liquid composition into the infusion bag or bottle containing the second aqueous composition is done in the temperature from about 20 °C to about 25 °C.

In the process of injecting the first liquid composition into the infusion bag or bottle containing the second aqueous composition, mixing (e.g., manual mixing such as with a stirrer) or agitation is not need. After the injection of the first liquid composition into the infusion bag or bottle is completed, the first liquid composition and second aqueous composition are mixed well (e.g., the bag containing the composition is gently inverted by hand) to obtain a transparent infusion solution free of precipitates. In some embodiments, the infusion bag or bottle containing the second aqueous composition is held still in the injection process. In some embodiments, the first liquid composition is injected undemeath the liquid surface of the second aqueous composition in the injection process. In some embodiments, after the injection is completed, the infusion bag or bottle is gently inverted repeatedly to mix well of the first liquid composition and second aqueous composition. In some embodiments, after the injection is completed, the infusion bag or bottle is gently inverted until a transparent solution free of precipitates is obtained. In some embodiments, after the injection is completed, the infusion bag or bottle is gently inverted for from about 5 seconds to about 10 minutes. In some embodiments, after the injection is completed, the infusion bag or bottle is gently inverted for from about 10 seconds to about 5 minutes. In some embodiments, after the injection is completed, the infusion bag or bottle is gently inverted for from about 0.5 minute to about 3 minutes. In some embodiments, after the injection is completed, the first liquid composition and second aqueous composition are mixed well until a transparent infusion solution free of precipitates is obtained.

After the first liquid composition and second aqueous composition are mixed well followed the injection of the first liquid composition into the infusion bag or bottle comprising the second aqueous composition, a transparent infusion solution free of precipitates comprising human serum albumin, docetaxel, or a pharmaceutically acceptable salt thereof, and ethanol in a parenterally acceptable vehicle is obtained. In some embodiments, the transparent infusion solution further comprises citric acid. In some embodiments, a parenterally acceptable vehicle is a normal saline solution or dextrose solution. In some embodiments, the transparent infusion solution stays transparent free of precipitates for at least 1 hour. In some embodiments, the transparent infusion solution stays transparent free of precipitates for at least 2 hours. In some embodiments, the transparent infusion solution stays transparent free of precipitates for at least 3 hours. In some embodiments, the transparent infusion solution stays transparent free of precipitates for at least 4 hours. In some embodiments, the transparent infusion solution stays transparent free of precipitates for at least 6 hours. In some embodiments, the transparent infusion solution stays transparent free of precipitates for at least 8 hours. In some embodiments, the transparent infusion solution stays transparent free of precipitates for at least 1 hour, 2 hours, 3 hours, 4 hours, or 6 hours, when the transparent solution keeps at from about 18 °C to about 25 °C. In some embodiments, the transparent infusion solution stays transparent free of precipitates for at least 1 hour, 2 hours, 3 hours, 4 hours, or 6 hours, when the transparent solution keeps at from about 20 °C to about 25 °C. In some embodiments, the transparent solution stays transparent free of precipitates for at least 2 hours, 4 hours, 6 hours, 8 hours, or 12 hours, when the transparent solution keeps at about 2-8 °C.

In some embodiments, the present disclosure provides methods of preparing the transparent parenteral infusion composition of docetaxel free of precipitates as described herein. A non-limiting preferred method is as follows.
1) Dissolve docetaxel and an acid (e.g. citric acid) in ethanol or a mixed solvent comprising ethanol (e.g., a mixed solvent of ethanol and PEG300, or ethanol and PEG400) to prepare the first liquid composition;
2) Add a human serum albumin solution (e.g. a clinically used 20%, 25%, or 5% solution of human serum albumin for infusion (w/v)) into an infusion bag or bottle containing a parenterally acceptable vehicle (e.g. a normal saline solution or dextrose solution) to prepare the second aqueous composition;
3) Inject the first liquid composition into the infusion bag or bottle containing the second aqueous composition; In the injection process, the infusion bag or bottle is held still and mixing or agitation is not need
4) After the injection of the first liquid composition into the infusion bag or bottle is completed, the first liquid composition and second aqueous composition are mixed well (e.g., the infusion bag or bottle containing the composition is gently inverted by hand) to obtain a transparent infusion solution free of precipitates.

One embodiment of the method is as follows, e.g., the method comprises:
1) dissolving docetaxel and an acid (e.g., citric acid) in ethanol or a mixed solvent comprising ethanol (e.g., a mixed solvent of ethanol and PEG300, or ethanol and PEG400) to prepare the first liquid composition (e.g., the method comprises obtaining a first liquid composition comprising a solution of docetaxel and an acid in ethanol or a mixed solvent comprising ethanol);
2) adding a human serum albumin solution (e.g. a clinically used 20%, 25%, or 5% solution of human serum albumin for infusion (w/v)) into an infusion bag or bottle containing a parenterally acceptable vehicle (e.g., a normal saline solution or dextrose solution) to prepare the second aqueous composition (e.g., the method comprises obtaining an infusion bag or bottle comprising a second aqueous composition comprising a solution of HSA and a parenterally acceptable vehicle);
3) injecting the first liquid composition into the infusion bag or bottle containing the second aqueous composition; in the injection process, the infusion bag or bottle is held still and mixing or agitation is not need (e.g., the method comprises combining the first liquid composition and the second aqueous composition by injecting the first liquid composition into the infusion bag of bottle comprising the second aqueous composition without mixing the compositions and/or without agitation of the infusion bag or bottle (e.g., by holding still the infusion bag or bottle)); and
4) after the injection of the first liquid composition into the infusion bag or bottle is completed, mixing well the first liquid composition and second aqueous composition (e.g., gently inverting by hand the infusion bag or bottle containing the composition) to obtain a transparent infusion solution free of precipitates (e.g., the method comprises agitating the infusion bag or bottle containing the composition to obtain the transparent infusion solution).

The first step and the second step of the preparation could be done in parallel or any different order.

Also, provided herein is a kit for the preparation of a parenteral infusion solution comprising: a first container containing a composition comprising docetaxel, or a pharmaceutically acceptable salt thereof; and a second container containing a composition comprising human serum albumin. In some embodiments, the kit comprises a first container containing a liquid composition comprising docetaxel, or a pharmaceutically acceptable salt thereof, and a second container containing a liquid composition comprising human serum albumin. In some embodiments, the kit comprises a first container containing a liquid composition comprising docetaxel, or a pharmaceutically acceptable salt thereof, and ethanol, and a second container containing a liquid composition comprising human serum albumin. In some embodiments, the kit comprises a first container containing a liquid composition comprising docetaxel, or a pharmaceutically acceptable salt thereof, citric acid, and ethanol, and a second container containing a liquid composition comprising human serum albumin. In some embodiments, the kit comprises a first container containing a liquid composition comprising docetaxel, or a pharmaceutically acceptable salt thereof, citric acid, and dehydrated ethanol, and a second container containing a liquid composition comprising human serum albumin. In some embodiments, the kit comprises a first container containing a liquid composition comprising docetaxel, or a pharmaceutically acceptable salt thereof, citric acid, and ethanol, and a second container containing a solution of human serum albumin for infusion (e.g. 20%, 25%, or 5% (w/v)).

In some embodiments, the first container does not comprise a lipid (e.g. soybean oil). In some embodiments, the lipid is soybean oil. In some embodiments, the first container does not comprise a surfactant. In some embodiments, the second container does not comprise a surfactant.

In some embodiments, ethanol contained in the first container is dehydrated ethanol. In some embodiments, the first container contains a liquid composition comprising docetaxel, or a pharmaceutically acceptable salt thereof, and ethanol. In some embodiments, the first container contains a liquid composition comprising docetaxel, or a pharmaceutically acceptable salt thereof, an acid, and ethanol. In some embodiments, the first container contains a liquid composition comprising docetaxel, or a pharmaceutically acceptable salt thereof, an organic acid, and ethanol. In some embodiments, the first container contains a liquid composition comprising docetaxel, or a pharmaceutically acceptable salt thereof, ethanol, and other organic solvents (e.g., an alcohol such as propylene glycol, or polyethylene glycol 300, etc.). In some embodiments, the first container contains a liquid composition comprising docetaxel, or a pharmaceutically acceptable salt thereof, ethanol, and propylene glycol. In some embodiments, the first container contains a liquid composition comprising docetaxel, or a pharmaceutically acceptable salt thereof, ethanol, and polyethylene glycol 300. In some embodiments, the first container contains a liquid composition comprising docetaxel, or a pharmaceutically acceptable salt thereof, and citric acid. In some embodiments, the first container contains a liquid composition comprising docetaxel, or a pharmaceutically acceptable salt thereof, citric acid, and ethanol. In some embodiments, the first container contains a liquid composition comprising docetaxel, or a pharmaceutically acceptable salt thereof, citric acid, and dehydrated ethanol. In some embodiments, the first container contains a liquid composition comprising docetaxel, or a pharmaceutically acceptable salt thereof, citric acid, ethanol, and propylene glycol. In some embodiments, ethanol in the first container is dehydrated ethanol. In some embodiments, the first container contains an ethanol solution containing docetaxel, or a pharmaceutically acceptable salt thereof. In some embodiments, the first container contains an ethanol solution containing docetaxel, or a pharmaceutically acceptable salt thereof, and citric acid. In some embodiments, the first container contains a dehydrated ethanol solution containing docetaxel, or a pharmaceutically acceptable salt thereof, and citric acid. In some embodiments, the first container contains an ethanol/propylene glycol solution containing docetaxel, or a pharmaceutically acceptable salt thereof, and citric acid. In some embodiments, the weight ratio of docetaxel, or a pharmaceutically acceptable salt thereof, and citric acid in the first container is from about 5000: 1 to about 1:1. In some embodiments, the weight ratio of docetaxel, or a pharmaceutically acceptable salt thereof, and citric acid in the first container is from about 2000:1 to about 1:1. In some embodiments, the weight ratio of docetaxel, or a pharmaceutically acceptable salt thereof, and citric acid in the first container is from about 1000:1 to about 1:1. In some embodiments, the weight ratio of docetaxel, or a pharmaceutically acceptable salt thereof, and citric acid in the first container is from about 500:1 to about 1:1. In some embodiments, the weight ratio of docetaxel, or a pharmaceutically acceptable salt thereof, and citric acid in the first container is from about 5000:1 to about 5:1. In some embodiments, the weight ratio of docetaxel, or a pharmaceutically acceptable salt thereof, and citric acid in the first container is from about 2000:1 to about 5:1. In some embodiments, the weight ratio of docetaxel, or a pharmaceutically acceptable salt thereof, and citric acid in the first container is from about 1000:1 to about 5:1. In some embodiments, the weight ratio of docetaxel, or a pharmaceutically acceptable salt thereof, and citric acid in the first container is from about 500:1 to about 5:1. In some embodiments, the weight ratio of docetaxel, or a pharmaceutically acceptable salt thereof, and citric acid in the first container is from about 5000: 1 to about 10:1. In some embodiments, the weight ratio of docetaxel, or a pharmaceutically acceptable salt thereof, and citric acid in the first container is from about 2000: 1 to about 10:1. In some embodiments, the weight ratio of docetaxel, or a pharmaceutically acceptable salt thereof, and citric acid in the first container is from about 1000:1 to about 10:1. In some embodiments, the weight ratio of docetaxel, or a pharmaceutically acceptable salt thereof, and citric acid in the first container is from about 500:1 to about 10:1. In some embodiments, the weight ratio of docetaxel, or a pharmaceutically acceptable salt thereof, and citric acid in the first container is from about 2000:1 to about 100:1. In some embodiments, the weight ratio of docetaxel, or a pharmaceutically acceptable salt thereof, and citric acid in the first container is from about 1000:1 to about 100:1. In some embodiments, the weight ratio of docetaxel, or a pharmaceutically acceptable salt thereof, and citric acid in the first container is from about 500:1 to about 100:1. In some embodiments, the weight ratio of docetaxel, or a pharmaceutically acceptable salt thereof, and citric acid in the first container is from about 5:1 to about 20:1. In some embodiments, the weight ratio of docetaxel, or a pharmaceutically acceptable salt thereof, and citric acid in the first container is about 5:1, about 10:1, about 15:1, or about 20:1. In some embodiments, the concentration of citric acid, or a pharmaceutically acceptable salt thereof, in the first container is from about 0.005 mg/ml to about 10 mg/ml. In some embodiments, the concentration of citric acid in the first container is from about 0.01 mg/ml to about 2 mg/ml. In some embodiments, the concentration of citric acid in the first container is from about 0.01 mg/ml to about 1 mg/ml. In some embodiments, the concentration of citric acid in the first container is from about 0.02 mg/ml to about 0.5 mg/ml. In some embodiments, the concentration of citric acid in the first container is from about 0.02 mg/ml to about 0.2 mg/ml.

In some embodiments, the amount of docetaxel contained in the first container is from about 10 mg to about 300 mg of docetaxel. In some embodiments, the amount of docetaxel contained in the first container is from about 20 mg to about 200 mg of docetaxel. In some embodiments, the amount of docetaxel contained in the first container is about 20 mg, about 40 mg, about 60 mg, about 80 mg, about 100 mg, about 120 mg, or about 160 mg of docetaxel. In some embodiments, the amount of docetaxel contained in the first container is about 80 mg or about 100 mg of docetaxel. In some embodiments, the amount of ethanol contained in the first container is from about 0.5 ml to about 50 ml of ethanol. In some embodiments, the amount of ethanol contained in the first container is from about 1 ml to about 20 ml of ethanol. In some embodiments, the amount of ethanol contained in the first container is from about 1 ml to about 10 ml of ethanol. In some embodiments, the amount of ethanol contained in the first container is from about 3 ml to about 6 ml of ethanol. In some embodiments, the amount of ethanol contained in the first container is from about 1 ml to about 5 ml of ethanol. In some embodiments, the amount of ethanol contained in the first container is from about 5 ml to about 10ml of ethanol. In some embodiments, the concentration of docetaxel in the first container is from about 1 mg/ml to about 200 mg/ml. In some embodiments, the concentration of docetaxel in the first container is from about 5 mg/ml to about 100 mg/ml. In some embodiments, the concentration of docetaxel in the first container is from about 10 mg/ml to about 50 mg/ml. In some embodiments, the concentration of docetaxel in the first container is about 20 mg/ml. In some embodiments, concentration of docetaxel in the first container is from about 10 mg/ml to about 30 mg/ml. In some embodiments, concentration of docetaxel in the first container is about 10 mg/ml, about 20 mg/ml, or about 30 mg/ml.

In some embodiments, the first container contains a solid composition comprising docetaxel, or a pharmaceutically acceptable salt thereof. In some embodiments, the first container contains a solid composition comprising docetaxel, or a pharmaceutically acceptable salt thereof, and an organic acid. In some embodiments, the first container contains a solid composition comprising docetaxel, or a pharmaceutically acceptable salt thereof, and citric acid.

In some embodiments, the second container contains a liquid composition comprising human serum albumin. In some embodiments, the second container contains a human serum albumin solution. In some embodiments, the second container contains a human serum albumin solution for infusion. In some embodiments, the second container contains a human serum albumin solution with the concentration of human serum albumin in the solution from about 1% to about 25% (w/v). In some embodiments, the second container contains a human serum albumin solution with the concentration of human serum albumin in the solution from about 5% to about 25% (w/v). In some embodiments, the second container contains a solution of human serum albumin for infusion. In some embodiments, the second container contains a 20% solution of human serum albumin for infusion (w/v). In some embodiments, the second container contains a 25% solution of human serum albumin for infusion (w/v). In some embodiments, the second container contains a 5% solution of human serum albumin for infusion (w/v). In some embodiments, the second container contains a liquid composition comprising from about 1 g to about 50 g of human serum albumin. In some embodiments, the second container contains a liquid composition comprising from about 1 g to about 30 g of human serum albumin. In some embodiments, the second container contains a liquid composition comprising from about 1 g to about 20 g of human serum albumin. In some embodiments, the second container contains a liquid composition comprising from about 1 g to about 10 g of human serum albumin. In some embodiments, the second container contains a liquid composition comprising from about 2 g to about 6 g of human serum albumin. In some embodiments, the second container contains a liquid composition comprising about 2 g, about 4 g, about 6 g, about 8 g, about 10 g, about 12 g, or about 16 g of human serum albumin.

In some embodiments, the first container comprises about 80mg docetaxel. In some embodiments, the first container comprises about 4ml ethanol. In some embodiments, the first container comprises about 80mg docetaxel and about 4ml ethanol.

In some embodiments, the kit comprises the first container comprising about 80mg docetaxel and the second container comprising from about 2g to about 8g of human serum albumin. In some embodiments, the kit comprises the first container comprising about 80mg docetaxel and the second container comprising from about 3g to about 6g of human serum albumin. In some embodiments, the kit comprises the first container comprising about 80mg docetaxel and the second container comprising from about 4g to about 5g of human serum albumin.

In some embodiments, the kit further comprises instructions to add the composition comprising docetaxel, or a pharmaceutically acceptable salt thereof, to the composition comprising human serum albumin.

The current marketed formulation of Taxotere comprises polysorbate 80 and ethanol. All other Docetaxel Injections currently marketed in the United States also contain polysorbate 80 and ethanol. The companies which market Doceteaxel Injections in the US include, not limiting to, HOSPIRA INC., SUN PHARMA, ACCORD HLTHCARE, SANDOZ, JIANGSU HENGRUI MED, ACTAVIS LLC, DR. REDDYS LABS LTD, TEIKOKU PHARMA, DFB ONCOLOGY LTD, INGENUS PHAMS LLC, MYLAN LABS INC, SHILPA MEDCARE LTD, AND AMNEAL, etc. Applicant surprisingly found that using the technical methods disclosed in the currently invention (e.g. adding/mixing clinically-used human serum albumin solution for infusion with normal saline in an infusion bag/bottle prior to adding (injecting) docetaxel injection into the infusion bag/bottle for preparation of infusion solution), there is actually no need to add polysorbate 80 in the formulation of docetaxel injection. In clinical use of the currently marketed Docetaxel injections, a large amount of Polysorbate 80 ( about 3-5g) has been given to patients in each infusion. Polysorbate 80 causes severe hypersensitivity reactions including fatal anaphylaxis to some patients. Thus Docetaxel injection free of polysorbate 80 presents a major clinical advantage in safety over the current marketed Docetaxel injections formulated with polysorbate 80.

Also, provided herein is an injectable pharmaceutical composition comprising docetaxel, or a pharmaceutically acceptable salt thereof, and ethanol, wherein the said injectable pharmaceutical composition comprises from about 5mg to 500mg of docetaxel, or a pharmaceutically acceptable salt thereof, wherein the said injectable pharmaceutical composition does not contain Polysorbate 80, and wherein the said injectable pharmaceutical composition is further mixed with an aqueous composition comprising human serum albumin to form a parenteral infusion solution prior to being infused or administered to patients. In some embodiments, the injectable pharmaceutical composition further comprises citric acid. In some embodiments, the mixing of the injectable pharmaceutical composition with the aqueous composition comprising human serum albumin is done in a infusion bag or bottle.

In some embodiments, the mixing of the injectable pharmaceutical composition with the aqueous composition comprising human serum albumin includes two steps. In the first step, the injectable pharmaceutical composition is injected into the infusion bag or bottle containing the aqueous composition comprising human serum albumin. In the injection process, the infusion bag or bottle is held still and mixing or agitation is not need. In the second step, After the injection of the injectable pharmaceutical composition into the infusion bag or bottle is completed, the injectable pharmaceutical composition and the aqueous composition are mixed well (e.g., the infusion bag or bottle containing the composition is gently inverted by hand) to obtain a transparent infusion solution free of precipitates.

In the process of injecting the injectable pharmaceutical composition into the infusion bag or bottle containing the aqueous composition comprising human serum albumin, the infusion bag or bottle is held still and mixing or agitation is not need in the injection process. We surprisingly found that by simply inverting the infusion bag or bottle gently (e.g. for 5-30 times), a transparent infusion solution free of precipitates is obtained.

In some embodiments, the parenteral infusion solution of docetaxel, or a pharmaceutically acceptable salt thereof, is a clear solution, not a nanoparticle suspension. In some embodiments, at least 30% docetaxel in the parenteral infusion solution is Free (unbound) docetaxel. In some embodiments, at least 40% docetaxel in the parenteral infusion solution is Free (unbound) docetaxel. In some embodiments, at least 50% docetaxel in the parenteral infusion solution is Free (unbound) docetaxel. In some embodiments, at least 60% docetaxel in the parenteral infusion solution is Free (unbound) docetaxel. In some embodiments, at least 70% docetaxel in the parenteral infusion solution is Free (unbound) docetaxel. Free (unbound) docetaxel means the fraction of docetaxel in the parenteral infusion solution that is not bound to human serum albumin. Concentration of free (unbound) docetaxel in the parenteral infusion solution is measured by ultrafiltration through a 30-kDa membrane. The experimental details of free (unbound) docetaxel measurement are described in the example section.

In some embodiments, provided herein is a doectaxel injection free of Polysorbate 80 comprising docetaxel and ethanol. In some embodiments, provided herein is a doectaxel injection free of Polysorbate 80 comprising docetaxel, citric acid, and ethanol. In some embodiments, provided herein is a doectaxel injection free of Polysorbate 80 consisting essentially of docetaxel, citric acid, and ethanol.

In some embodiments, the injectable pharmaceutical composition comprises from about 10mg to about 300mg of docetaxel, or a pharmaceutically acceptable salt thereof. In some embodiments, the injectable pharmaceutical composition comprises from about 20mg to about 200mg of docetaxel, or a pharmaceutically acceptable salt thereof. In some embodiments, the injectable pharmaceutical composition comprises 20mg, 40mg, 80mg, 120mg, or 160mg docetaxel. In some embodiments, the injectable pharmaceutical composition comprises about 20mg, about 40mg, about 80mg, about 120mg, or about 160mg docetaxel.

In some embodiments, the injectable pharmaceutical composition does not comprise a lipid (e.g. soybean oil). In some embodiments, the injectable pharmaceutical composition does not comprise a surfactant. In some embodiments, the injectable pharmaceutical composition contains one or more other organic solvents (e.g. propylene glycol, polyethylene glycol 300, polyethylene glycol 400, etc.) in addition to ethanol. In some embodiments, the injectable pharmaceutical composition contains more than 50% ethanol (v/v). In some embodiments, the injectable pharmaceutical composition contains more than 70% ethanol (v/v). In some embodiments, the injectable pharmaceutical composition contains more than 80% ethanol (v/v).

In some embodiments, the concentration of docetaxel, or a pharmaceutically acceptable salt thereof, in the injectable pharmaceutical composition is from about 5mg/ml to about 40mg/ml. In some embodiments, the concentration of docetaxel, or a pharmaceutically acceptable salt thereof, in the injectable pharmaceutical composition is from about 10mg/ml to about 20mg/ml. In some embodiments, the concentration of docetaxel, or a pharmaceutically acceptable salt thereof, in the injectable pharmaceutical composition is about 10mg/ml, 15mg/ml, or about 20mg/ml.

The injectable pharmaceutical composition further comprises an organic acid. The most preferred acid for use in accordance with the present invention is citric acid.

In some embodiments, pH value of the injectable pharmaceutical composition is from about 3 to about 9. In some embodiments, pH value of the injectable pharmaceutical composition is from about 3 to about 8. In some embodiments, pH value of the injectable pharmaceutical composition is from about 3 to about 7. In some embodiments, pH value of the injectable pharmaceutical composition is from about 3 to about 6.5. In some embodiments, pH value of the injectable pharmaceutical composition is from about 3.5 to about 6.5. In some embodiments, pH value of the injectable pharmaceutical composition is from about 3.5 to about 6. In some embodiments, pH value of the injectable pharmaceutical composition is from about 3.5 to about 5.5. In some embodiments, pH value of the injectable pharmaceutical composition is from about 4 to about 5. To measure the pH value of the injectable pharmaceutical dosage form, the injectable pharmaceutical dosage form is mixed with normal saline in 1:1 ratio (v/v) to obtain an aqueous solution, and then pH value of the aqueous solution is tested.

According to yet another embodiment, the injectable pharmaceutical composition of docetaxel is formulated into an injectable pharmaceutical dosage form. In some embodiments, the injectable pharmaceutical dosage form comprises about 20 mg, about 40 mg, about 80 mg, about 120 mg, or about 160 mg of docetaxel, or a pharmaceutically acceptable salt thereof, citric acid and ethanol, wherein the concentration of docetaxel, or a pharmaceutically acceptable salt thereof, in the injectable pharmaceutical dosage form is from 5 mg/ml to 40 mg/ml (e.g., about 20 mg/ml, about 10 mg/ml). In some embodiments, the injectable pharmaceutical dosage form has pH value from about 3 to about 6.5. The preferred pH of the injectable pharmaceutical dosage form is from about 3.5 to about 5.5.

Also, provided herein is a formulation for use in a method of treating cancer, the method comprising administering to a subject in need thereof a therapeutically effective amount of any composition or formulation described herein. In some embodiments, the method comprises parenterally administering to a subject in need thereof of a therapeutically effective amount of a liquid infusion composition of the present disclosure.

In some embodiments, the cancer is selected from the group consisting of bladder cancer, brain cancer, breast cancer, colorectal cancer, cervical cancer, gastrointestinal cancer, genitourinary cancer, head and neck cancer, lung cancer, ovarian cancer, pancreatic cancer, prostate cancer, renal cancer, skin cancer, and testicular cancer.

In some embodiments, cancer is selected from sarcoma, angiosarcoma, fibrosarcoma, rhabdomyosarcoma, liposarcoma, myxoma, rhabdomyoma, fibroma, lipoma, teratoma, non-small cell lung cancer (NSCLC), bronchogenic carcinoma squamous cell, undifferentiated small cell, undifferentiated large cell, adenocarcinoma, alveolar bronchiolar carcinoma, bronchial adenoma, sarcoma, lymphoma, chondromatous hamartoma, mesothelioma, gastrointestinal cancer, cancer of the esophagus, squamous cell carcinoma, adenocarcinoma, leiomyosarcoma, lymphoma, cancer of the stomach, carcinoma, lymphoma, leiomyosarcoma, cancer of the pancreas, ductal adenocarcinoma, insulinoma, glucagonoma, gastrinoma, carcinoid tumor, vipoma, cancer of the small bowel, adenocarcinoma, lymphoma, carcinoid tumors, Kaposi's sarcoma, leiomyoma, hemangioma, lipoma, neurofibroma, fibroma, cancer of the large bowel or colon, tubular adenoma, villous adenoma, hamartoma, leiomyoma, genitourinary tract cancer , cancer of the kidney adenocarcinoma, Wilm's tumor (nephroblastoma), lymphoma, leukemia, cancer of the bladder, cancer of the urethra, squamous cell carcinoma, transitional cell carcinoma, cancer of the prostate, cancer of the testis, seminoma, teratoma, embryonal carcinoma, teratocarcinoma, choriocarcinoma, sarcoma, interstitial cell carcinoma, fibroma, fibroadenoma, adenomatoid tumors, lipoma, liver cancer, hepatoma hepatocellular carcinoma, cholangiocarcinoma, hepatoblastoma, angiosarcoma, hepatocellular adenoma, hemangioma, bone cancer, osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor, chordoma, osteochrondroma (osteocartilaginous exostoses), benign chondroma, chondroblastoma, chondromyxofibroma, osteoid osteoma giant cell tumor, nervous system cancer, cancer of the skull, osteoma, hemangioma, granuloma, xanthoma, osteitis deformans, cancer of the meninges meningioma, meningiosarcoma, gliomatosis, cancer of the brain, astrocytoma, medulloblastoma, glioma, ependymoma, germinoma (pinealoma), glioblastoma multiforme, oligodendroglioma, schwannoma, retinoblastoma, congenital tumors, cancer of the spinal cord, neurofibroma, meningioma, glioma, sarcoma, gynecological cancer, cancer of the uterus, endometrial carcinoma, cancer of the cervix, cervical carcinoma, pre tumor cervical dysplasia, cancer of the ovaries, ovarian carcinoma, serous cystadenocarcinoma, mucinous cystadenocarcinoma, unclassified carcinoma, granulosa-theca cell tumor, Sertoli Leydig cell tumor, dysgerminoma, malignant teratoma, cancer of the vulva, squamous cell carcinoma, intraepithelial carcinoma, adenocarcinoma, fibrosarcoma, melanoma, cancer of the vagina, clear cell carcinoma, squamous cell carcinoma, botryoid sarcoma, embryonal rhabdomyosarcoma, cancer of the fallopian tubes, hematologic cancer, cancer of the blood, acute myeloid leukemia (AML), chronic myeloid leukemia (CML), acute lymphoblastic leukemia (ALL), chronic lymphoblastic leukemia, chronic lymphocytic leukemia, myeloproliferative diseases, multiple myeloma, myelodysplastic syndrome, Hodgkin's lymphoma, non-Hodgkin's lymphoma (malignant lymphoma), Waldenstrom's macroglobulinemia, skin cancer, malignant melanoma, basal cell carcinoma, squamous cell carcinoma, Kaposi's sarcoma, moles dysplastic nevi, lipoma, angioma, dermatofibroma, keloids, psoriasis, adrenal gland cancer, and neuroblastoma.

In some embodiments, the cancer is selected from breast cancer, non-small cell lung cancer, hormone refractory prostate cancer, gastric adenocarcinoma, and squamous cell carcinoma of head and neck cancer.

Also, provided herein a parenteral pharmaceutical formulation comprising two compositions that are mixed prior to being infused or administered to patients, that comprises: (a) a first liquid composition comprising a substantially water-insoluble pharmaceutically active agent, or a pharmaceutically acceptable salt thereof, and (b) a second aqueous composition comprising human serum albumin and a parenterally acceptable vehicle, and wherein the substantially water-insoluble pharmaceutically active agent is the API (active pharmaceutical ingredient) of the said parenteral pharmaceutical formulation. In some embodiments, the first liquid composition comprises a substantially water-insoluble pharmaceutically active agent, or a pharmaceutically acceptable salt thereof, and an organic solvent (e.g., alcohol or any other suitable solvent such as DMSO). In some embodiments, the first liquid composition comprises a substantially water-insoluble pharmaceutically active, or a pharmaceutically acceptable salt thereof, agent and an alcohol. In some embodiments, the first liquid composition comprises a substantially water-insoluble pharmaceutically active agent, or a pharmaceutically acceptable salt thereof, and ethanol. In some embodiments, the mixing of the first liquid composition and the second aqueous composition is done in a infusion bag or bottle. In some embodiments, the second aqueous composition is contained in a infusion bag or bottle. In some embodiments, the first liquid composition is injected into a infusion bag or bottle which contains the second aqueous composition. In some embodiments, the mixing of the first liquid composition and the second aqueous composition is done in a hospital or clinic prior to being infused or administered to patients.

As used herein, the term "water-insoluble" refers to the limited solubility of a pharmacologically active agent, or a pharmaceutically acceptable salt thereof, in aqueous solutions (such as water, physiological saline, injectable dextrose solutions, etc). The United States Pharmacoepia/National Formulary (USP/NF) generally expresses solubility in terms of the volume of solvent required to dissolve 1 gram of the pharmacologically active agent at a specified temperature (e.g., 1 g aspirin in 300 mL water or 5 mL ethanol at 25 °C.). Other references may use more subjective terms to describe solubility, such as those given in the following table from Remington's Pharmaceutical Sciences, Mack Publishing Co., Easton, Pa., by Joseph Remington and Alfonso Gennaro: Mack Publishing, 1995,

**Table 1**

| Descriptive terms | Parts of solvent needed for 1 part solute |
|---|---|
| Very soluble | <1 |
| Freely soluble | 1-10 |
| Soluble | 10-30 |
| Sparingly soluble | 30-100 |
| Slightly soluble | 100-1000 |
| Very slightly soluble | 1000-10,000 |
| Practically insoluble or insoluble | >10,000 |

As used herein, the term "substantially water-insoluble pharmaceutically active agent" means those agents that are sparingly, slightly, or very slightly soluble, or practically insoluble or insoluble according to the definitions for solubility provided in Table 1 above, or those agents that have solubility less than 1 mg/ml in water. For example, the substantially water-insoluble pharmaceutically active agent can be docetaxel or paclitaxel, or a pharmaceutically acceptable salt thereof.

In some embodiments, the substantially water-insoluble pharmaceutically active agent, or a pharmaceutically acceptable salt thereof, has a solubility of less than about 1 mg/ml, about 0.9 mg/mL, about 0.8 mg/mL, about 0.7 mg/mL, about 0.6 mg/mL, about 0.5 mg/mL, about 0.4 mg/mL, about 0.3 mg/mL, about 0.2 mg/mL, about 0.1 mg/mL, about 0.09 mg/mL, about 0.08 mg/mL, about 0.07 mg/mL, about 0.06 mg/mL, about 0.05 mg/mL, about 0.04 mg/mL, about 0.03 mg/mL, about 0.02 mg/mL, or less than about 0.01 mg/mL.

In some embodiments, the said pharmaceutical formulation does not comprise a lipid (e.g. soybean oil). In some embodiments, the first liquid composition does not comprise a lipid (e.g. soybean oil). In some embodiments, the lipid is soybean oil.

In some embodiments, the said pharmaceutical formulation does not comprise a surfactant. In some embodiments, the first liquid composition does not comprise a surfactant. In some embodiments, the second aqueous composition does not comprise a surfactant.

### EXAMPLES

### Example 1:

300.8 mg of docetaxel and 60.9 mg of citric acid were dissolved in 15 ml of dehydrated ethanol to obtain a docetaxel API solution. 25 ml of a 20% solution of human serum albumin for infusion was added into a 250 ml infusion bottle containing 150 ml normal saline solution (0.5% NaCl solution). After the albumin solution was mixed well with saline solution, the infusion bottle was kept still at horizontal position, and 2.5 ml of the docetaxel API solution was injected rapidly into the infusion bottle. Then the solution was mixed well to obtain a transparent solution free of precipitates. The pH of the obtained transparent solution is 6.78. The particulate matter of the transparent solution was also measured. 1 ml of the transparent solution has about 44 particles that are greater than 10 µm in size and about 3 particles that are greater than 25 µm in size. The solution was then divided into two portions. The one portion of the transparent solution was kept at 20 °C to 25 °C, which stayed as a transparent solution free of precipitates for 3 hours. The other portion of the transparent solution was kept at 2 °C to 8 °C for 2 hours, which stayed as a transparent solution free of precipitates. After 2 hours, the solution was kept at 20 °C to 25 °C, which stayed as a transparent solution free of precipitates for additional 2 hours.

30 ml of a 20% solution of human serum albumin for infusion was added into a 250 ml infusion bottle containing 180 ml normal saline solution (0.5% NaCl solution). After the albumin solution was mixed well with saline solution, the infusion bottle was kept still at horizontal position, and 3 ml of the docetaxel API solution was injected rapidly into the infusion bottle. Then the solution was mixed well to obtain a transparent solution free of precipitates. The pH of the obtained transparent solution is 6.77. 1 ml of the particulate matter of the transparent solution was also measured. The transparent solution has about 86 particles that are greater than 10 µm in size and about 5 particles that are greater than 25 µm in size. The solution was then divided into two portions. The one portion of the transparent solution was kept at 20 °C to 25 °C, which stayed as a transparent solution free of precipitates for 3 hours. The other portion of the transparent solution was kept at 2 °C to 8 °C for 2 hours, which stayed as a transparent solution free of precipitates. After 2 hours, the solution was kept at 20 °C to 25 °C, which stayed as a transparent solution free of precipitates for additional 2 hours.

### Example 2:

300.8 mg of docetaxel and 59.6 mg of citric acid were dissolved in 15 ml of dehydrated ethanol to obtain a docetaxel API solution. 20 ml of a 20% solution of human serum albumin for infusion was added into a 250 ml infusion bottle containing 120 ml normal saline solution (0.5% NaCl solution). After the albumin solution was mixed well with saline solution, the infusion bottle was kept still at horizontal position, and 2 ml of the docetaxel API solution was injected rapidly into the infusion bottle. Then the solution was mixed well to obtain a transparent solution free of precipitates. The particulate matter of the transparent solution was measured. 1 ml of the transparent solution has about 96 particles that are greater than 10 µm in size and about 6 particles that are greater than 25 µm in size. The solution was then divided into two portions. The one portion of the transparent solution was kept at 20 °C to 25 °C, which stayed as a transparent solution free of precipitates for 2 hours. The other portion of the transparent solution was kept at 2 °C to 8 °C for 4 hours, which stayed as a transparent solution free of precipitates. After 4 hours, the solution was kept at 20 °C to 25 °C, which stayed as a transparent solution free of precipitates for additional 2 hours.

15 ml of a 20% solution of human serum albumin for infusion was added into a 250 ml infusion bottle containing 90 ml normal saline solution (0.5% NaCl solution). After the albumin solution was mixed well with saline solution, the infusion bottle was kept still at horizontal position, and 1.5 ml of the docetaxel API solution was injected rapidly into the infusion bottle. Then the solution was mixed well to obtain a transparent solution free of precipitates. 1 ml of the particulate matter of the transparent solution was measured. The transparent solution has about 49 particles that are greater than 10 µm in size and about 2 particles that are greater than 25 µm in size. The solution was then divided into two portions. The one portion of the transparent solution was kept at 20°C to 25 °C, which stayed as a transparent solution free of precipitates for 4 hours. The other portion of the transparent solution was kept at 2 °C to 8 °C for 2 hours, which stayed as a transparent solution free of precipitates. After 2 hours, the solution was kept at 20°C to 25 °C, which stayed as a transparent solution free of precipitates for additional 1 hour.

### Example 3:

1.0005 g of docetaxel and 199.8 mg of citric acid were dissolved in 50 ml of dehydrated ethanol to obtain a docetaxel API solution. 50 ml of a 20% solution of human serum albumin for infusion was added into a 500 ml infusion bottle containing 300 ml normal saline solution (0.5% NaCl solution). After the albumin solution was mixed well with saline solution, the infusion bottle was kept still at horizontal position, and 5 ml of the docetaxel API solution was injected rapidly into the infusion bottle. Then the solution was mixed well to obtain a transparent solution free of precipitates. The particulate matter of the transparent solution was measured. 1 ml of the transparent solution has about 39 particles that are greater than 10 µm in size and about 2 particles that are greater than 25 µm in size. The solution was then divided into three portions. The one portion of the transparent solution was kept at 20 °C to 25 °C, which stayed as a transparent solution free of precipitates for 4 hours. The second portion of the transparent solution was kept at 2 °C to 8 °C for 7 hours, which stayed as a transparent solution free of precipitates. The third portion of the transparent solution was kept at 2 °C to 8 °C for 4 hours, which stayed as a transparent solution free of precipitates. After 4 hours, the solution was kept at 20 °C to 25 °C, which stayed as a transparent solution free of precipitates for additional 2.5 hours.

### Example 4:

300 mg of docetaxel and 59.5 mg of citric acid were dissolved in 15 ml of dehydrated ethanol to obtain a docetaxel API solution. 65 ml of a 20% solution of human serum albumin for infusion was added into a 500 ml infusion bottle containing 390 ml normal saline solution (0.5% NaCl solution). After the albumin solution was mixed well with saline solution, the infusion bottle was kept still at horizontal position, and 6.5 ml of the docetaxel API solution was injected rapidly into the infusion bottle. Then the solution was mixed well to obtain a transparent solution free of precipitates. The pH of the obtained transparent solution is 6.78. The particulate matter of the transparent solution was also measured. 1 ml of the transparent solution has about 53 particles that are greater than 10 µm in size and about 5 particles that are greater than 25 µm in size. The solution was then divided into three portions. The one portion of the transparent solution was kept at 20 °C to 25 °C, which stayed as a transparent solution free of precipitates for 2 hours. The second portion of the transparent solution was kept at 2 °C to 8 °C for 3 hours, which stayed as a transparent solution free of precipitates. After 3 hours, the solution was kept at 20 °C to 25 °C, which stayed as a transparent solution free of precipitates for additional 1.5 hours. The third portion of the transparent solution was kept at 2 °C to 8 °C for 4 hours, which stayed as a transparent solution free of precipitates. After 4 hours, the solution was kept at 20 °C to 25 °C, which stayed as a transparent solution free of precipitates for additional 2 hours.

### Example 5:

300.2 mg of docetaxel and 30.1 mg of citric acid were dissolved in 15 ml of dehydrated ethanol to obtain a docetaxel API solution. 19 ml of a 20% solution of human serum albumin for infusion was added into a 250 ml infusion bottle containing 120 ml normal saline solution (0.5% NaCl solution). After the albumin solution was mixed well with saline solution, the infusion bottle was kept still at horizontal position, and 2 ml of the docetaxel API solution was injected rapidly into the infusion bottle. Then the solution was mixed well to obtain a transparent solution free of precipitates. The particulate matter of the transparent solution was measured. 1 ml of the transparent solution has about 64 particles that are greater than 10 µm in size and about 2 particles that are greater than 25 µm in size. The solution was then divided into two portions. The one portion of the transparent solution was kept at 20 °C to 25 °C, which stayed as a transparent solution free of precipitates for 3.5 hours. The other portion of the transparent solution was kept at 2 °C to 8 °C for 4 hours, which stayed as a transparent solution free of precipitates. After 4 hours, the solution was kept at 20 °C to 25 °C, which stayed as a transparent solution free of precipitates for additional 1.5 hours.

18 ml of a 20% solution of human serum albumin for infusion was added into a 250 ml infusion bottle containing 120 ml normal saline solution (0.5% NaCl solution). After the albumin solution was mixed well with saline solution, the infusion bottle was kept still at horizontal position, and 2 ml of the docetaxel API solution was injected rapidly into the infusion bottle. Then the solution was mixed well to obtain a transparent solution free of precipitates. 1 ml of the particulate matter of the transparent solution was measured. The transparent solution has about 102 particles that are greater than 10 µm in size and about 2 particles that are greater than 25 µm in size. The solution was then divided into two portions. The one portion of the transparent solution was kept at 20 °C to 25 °C, which stayed as a transparent solution free of precipitates for 1.5 hours. The other portion of the transparent solution was kept at 2 °C to 8 °C for 4 hours, which stayed as a transparent solution free of precipitates. After 4 hours, the solution was kept at 20 °C to 25 °C, which stayed as a transparent solution free of precipitates for additional 2 hours.

65 ml of a 20% solution of human serum albumin for infusion was added into a 500 ml infusion bottle containing 390 ml normal saline solution (0.5% NaCl solution). After the albumin solution was mixed well with saline solution, the infusion bottle was kept still at horizontal position, and 6.5 ml of the docetaxel API solution was injected rapidly into the infusion bottle. Then the solution was mixed well to obtain a transparent solution free of precipitates. The particulate matter of the transparent solution was measured. 1 ml of the transparent solution has about 51 particles that are greater than 10 µm in size and about 2 particles that are greater than 25 µm in size. The solution was then divided into three portions. The one portion of the transparent solution was kept at 20 °C to 25 °C, which stayed as a transparent solution free of precipitates for 3 hours. The second portion of the transparent solution was kept at 2 °C to 8 °C for 4 hours, which stayed as a transparent solution free of precipitates. After 4 hours, the solution was kept at 20 °C to 25 °C, which stayed as a transparent solution free of precipitates for additional 3 hours. The third portion of the transparent solution was kept at 2 °C to 8 °C for 6 hours, which stayed as a transparent solution free of precipitates. After 6 hours, the solution was kept at 20 °C to 25 °C, which stayed as a transparent solution free of precipitates for additional 2 hours.

### Example 6:

100.3 mg of docetaxel and 5.1 mg of citric acid were dissolved in 5 ml of dehydrated ethanol to obtain a docetaxel API solution. 20 ml of a 20% solution of human serum albumin for infusion was added into a 250 ml infusion bottle containing 120 ml normal saline solution (0.5% NaCl solution). After the albumin solution was mixed well with saline solution, the infusion bottle was kept still at horizontal position, and 2 ml of the docetaxel API solution was injected rapidly into the infusion bottle. Then the solution was mixed well to obtain a transparent solution free of precipitates. The particulate matter of the transparent solution was measured. 1 ml of the transparent solution has about 63 particles that are greater than 10 µm in size and about 1 particle that is greater than 25 µm in size. The solution was then divided into two portions. The one portion of the transparent solution was kept at 20 °C to 25 °C, which stayed as a transparent solution free of precipitates for 4.5 hours. The other portion of the transparent solution was kept at 2 °C to 8 °C for 6 hours, which stayed as a transparent solution free of precipitates. After 6 hours, the solution was kept at 20 °C to 25 °C, which stayed as a transparent solution free of precipitates for additional 1.5 hours.

### Example 7:

20 mg citric acid was dissolved in 20 ml dehydrated ethanol to obtain an citric acid solution (1mg/ml). 100.4 mg of docetaxel was dissolved in 3 ml of dehydrated ethanol, followed by adding 2 ml of the citric acid solution (1 mg/ml) to obtain a docetaxel API solution. 19 ml of a 20 % solution of human serum albumin for infusion was added into a 250 ml infusion bag containing 120 ml normal saline solution (0.5% NaCl solution). After the albumin solution was mixed well with saline solution, the infusion bag was kept still at horizontal position, and 2 ml of the docetaxel API solution was injected rapidly into the infusion bag. Then the solution was mixed well to obtain a transparent solution free of precipitates. The pH of the obtained transparent solution is 7.05. The particulate matter of the transparent solution was also measured. 1 ml of the transparent solution has about 66 particles that are greater than 10 µm in size and about 1 particle that is greater than 25 µm in size. The solution was then divided into two portions. The one portion of the transparent solution was kept at 20 °C to 25 °C, which stayed as a transparent solution free of precipitates for 3 hours. The other portion of the transparent solution was kept at 2 °C to 8 °C for 4 hours, which stayed as a transparent solution free of precipitates. After 4 hours, the solution was kept at 20 °C to 25 °C, which stayed as a transparent solution initially and became cloudy with precipitation in about 1 hour.

### Example 8:

19.8 mg citric acid was dissolved in 20 ml dehydrated ethanol to obtain an citric acid solution. 200 mg of docetaxel was dissolved in 9 ml of dehydrated ethanol, followed by adding 1 ml of the prepared citric acid solution to obtain a docetaxel API solution. 65 ml of a 20% solution of human serum albumin for infusion was added into a 500 ml infusion bag containing 390 ml normal saline solution (0.5% NaCl solution). After the albumin solution was mixed well with saline solution, the infusion bag was kept still at horizontal position, and 6.5 ml of the docetaxel API solution was injected rapidly into the infusion bag. Then the solution was mixed well to obtain a transparent solution free of precipitates. The pH of the obtained transparent solution is 6.95. The particulate matter of the transparent solution was also measured. 1 ml of the transparent solution has about 29 particles that are greater than 10 µm in size and about 2 particles that are greater than 25 µm in size. The solution was then divided into three portions. The one portion of the transparent solution was kept at 20 °C to 25 °C, which stayed as a transparent solution free of precipitates for 3 hours. The second portion of the transparent solution was kept at 2 °C to 8 °C for 4 hours, which stayed as a transparent solution free of precipitates. After 4 hours, the solution was kept at 20 °C to 25 °C, which stayed as a transparent solution free of precipitates for additional 0.5 hour. The third portion of the transparent solution was kept at 2 °C to 8 °C for 6 hours, which stayed as a transparent solution free of precipitates . After 6 hours, the solution was kept at 20 °C to 25 °C, which stayed as a transparent solution free of precipitates for additional 1.5 hours.

100.1 mg of docetaxel was dissolved in 2.5 ml of dehydrated ethanol, followed by adding 0.5 ml of the prepared citric acid solution to obtain a docetaxel API solution. 20 ml of a 20% solution of human serum albumin for infusion was added into a 250 ml infusion bag containing 120 ml normal saline solution (0.5% NaCl solution). After the albumin solution was mixed well with saline solution, the infusion bag was kept still at horizontal position, and 1.2 ml of the docetaxel API solution was injected rapidly into the infusion bag. Then the solution was mixed well to obtain a transparent solution free of precipitates. The pH of the obtained transparent solution is 6.99. The particulate matter of the transparent solution was also measured. 1 ml of the transparent solution has about 64 particles that are greater than 10 µm in size and about 1 particle that is greater than 25 µm in size. The solution was then divided into two portions. The one portion of the transparent solution was kept at 20 °C to 25 °C, which stayed as a transparent solution free of precipitates for 4 hours. The other portion of the transparent solution was kept at 2 °C to 8 °C for 6 hours, which stayed as a transparent solution free of precipitates. After 6 hours, the solution was kept at 20 °C to 25 °C, which stayed as a transparent solution initially and became cloudy with precipitation in about 0.5 hour.

100.4 mg of docetaxel was dissolved in 3.5 ml of dehydrated ethanol, followed by adding 0.5 ml of the prepared citric acid solution and 1 ml propylene glycol to obtain a docetaxel API solution. 20 ml of a 20% solution of human serum albumin for infusion was added into a 250 ml infusion bag containing 120 ml normal saline solution (0.5% NaCl solution). After the albumin solution was mixed well with saline solution, the infusion bag was kept still at horizontal position, and 2 ml of the docetaxel API solution was injected rapidly into the infusion bag. Then the solution was mixed well to obtain a transparent solution free of precipitates. The particulate matter of the transparent solution was measured. 1 ml of the transparent solution has about 32 particles that are greater than 10 µm in size and about 1 particle that is greater than 25 µm in size. The solution was then divided into two portions. The one portion of the transparent solution was kept at 20 °C to 25 °C, which stayed as a transparent solution free of precipitates for 5 hours. The other portion of the transparent solution was kept at 2 °C to 8 °C for 6 hours, which stayed as a transparent solution free of precipitates. After 6 hours, the solution was kept at 20 °C to 25 °C, which stayed as a transparent solution free of precipitates for additional 2 hours.

### Example 9:

20 mg citric acid was dissolved in 20 ml dehydrated ethanol to obtain an citric acid solution. 300.9 mg of docetaxel was dissolved in 14.4 ml of dehydrated ethanol, followed by adding 0.6 ml of the prepared citric acid solution to obtain a docetaxel API solution. 18 ml of a 20% solution of human serum albumin for infusion was added into a 250 ml infusion bag containing 120 ml normal saline solution (0.5% NaCl solution). After the albumin solution was mixed well with saline solution, the infusion bag was kept still at horizontal position, and 2 ml of the docetaxel API solution was injected rapidly into the infusion bag. Then the solution was mixed well to obtain a transparent solution free of precipitates. The pH of the obtained transparent solution is 6.96. The particulate matter of the transparent solution was also measured. 1 ml of the transparent solution has about 49 particles that are greater than 10 µm in size and about 6 particles that are greater than 25 µm in size. The solution was then divided into two portions. The one portion of the transparent solution was kept at 20 °C to 25 °C, which stayed as a transparent solution for 4.5 hours free of precipitates. The other portion of the transparent solution was kept at 2 °C to 8 °C for 6 hours, which stayed as a transparent solution free of precipitates. After 6 hours, the solution was kept at 20 °C to 25 °C, which stayed as a transparent solution free of precipitates for additional 1 hour.

20 ml of a 20% solution of human serum albumin for infusion was added into a 250 ml infusion bag containing 120 ml normal saline solution (0.5% NaCl solution). After the albumin solution was mixed well with saline solution, the infusion bag was kept still at horizontal position, and 2 ml of the docetaxel API solution was injected rapidly into the infusion bag. Then the solution was mixed well to obtain a transparent solution free of precipitates. The pH of the obtained transparent solution is 6.96. The particulate matter of the transparent solution was also measured. 1 ml of the transparent solution has about 55 particles that are greater than 10 µm in size and about 3 particles that are greater than 25 µm in size. The solution was then divided into two portions. The one portion of the transparent solution was kept at 20 °C to 25 °C, which stayed as a transparent solution free of precipitates for 4 hours. The other portion of the transparent solution was kept at 2 °C to 8 °C for 6 hours, which stayed as a transparent solution free of precipitates. After 6 hours, the solution was kept at 20 °C to 25 °C, which stayed as a transparent solution initially and became cloudy with precipitation in about 0.5 hour.

35 ml of a 20% solution of human serum albumin for infusion was added into a 500 ml infusion bag containing 210 ml normal saline solution (0.5% NaCl solution). After the albumin solution was mixed well with saline solution, the infusion bag was kept still at horizontal position, and 3.5 ml of the docetaxel API solution was injected rapidly into the infusion bag. Then the solution was mixed well to obtain a transparent solution free of precipitates. The pH of the obtained transparent solution is 6.96. The particulate matter of the transparent solution was also measured. 1 ml of the transparent solution has about 83 particles that are greater than 10 µm in size and about 7 particles that are greater than 25 µm in size. The solution was then divided into three portions. The one portion of the transparent solution was kept at 20 °C to 25 °C, which stayed as a transparent solution free of precipitates for 3.5 hours. The second portion of the transparent solution was kept at 2 °C to 8 °C for 4 hours, which stayed as a transparent solution free of precipitates. After 4 hours, the solution was kept at 20 °C to 25 °C, which stayed as a transparent solution free of precipitates for additional 1 hour. The third portion of the transparent solution was kept at 2 °C to 8 °C for 6 hours, which stayed as a transparent solution free of precipitates. After 6 hours, the solution was kept at 20 °C to 25 °C, which stayed as a transparent solution free of precipitates for additional 1 hour.

50 ml of a 20% solution of human serum albumin for infusion was added into a 500 ml infusion bag containing 300 ml normal saline solution (0.5% NaCl solution). After the albumin solution was mixed well with saline solution, the infusion bag was kept still at horizontal position, and 5 ml of the docetaxel API solution was injected rapidly into the infusion bag. Then the solution was mixed well to obtain a transparent solution free of precipitates. The pH of the obtained transparent solution is 6.96. The particulate matter of the transparent solution was also measured. 1 ml of the transparent solution has about 95 particles that are greater than 10 µm in size and about 3 particles that are greater than 25 µm in size. The solution was then divided into three portions. The one portion of the transparent solution was kept at 20 °C to 25 °C, which stayed as a transparent solution free of precipitates for 4 hours. The second portion of the transparent solution was kept at 2 °C to 8 °C for 4 hours, which stayed as a transparent solution free of precipitates. After 4 hours, the solution was kept at 20 °C to 25 °C, which stayed as a transparent solution free of precipitates for additional 1 hour. The third portion of the transparent solution was kept at 2 °C to 8 °C for 6 hours, which stayed as a transparent solution free of precipitates. After 6 hours, the solution was kept at 20 °C to 25 °C, which stayed as a transparent solution free of precipitates for additional 1.5 hours.

### Example 10:

19.8 mg citric acid was dissolved in 20 ml dehydrated ethanol to obtain an citric acid solution. 299.5 mg of docetaxel was dissolved in 14.4 ml of dehydrated ethanol, followed by adding 0.6 ml of the prepared citric acid solution to obtain a docetaxel API solution. 17.5 ml of a 20% solution of human serum albumin for infusion was added into a 250 ml infusion bag containing 105 ml normal saline solution (0.5% NaCl solution). After the albumin solution was mixed well with saline solution, the infusion bag was kept still at horizontal position, and 1.75 ml of the docetaxel API solution was injected rapidly into the infusion bag. Then the solution was mixed well to obtain a transparent solution free of precipitates. The pH of the obtained transparent solution is 6.96. The particulate matter of the transparent solution was also measured. 1 ml of the transparent solution has about 57 particles that are greater than 10 µm in size and about 1 particle that is greater than 25 µm in size. The transparent solution was kept at 20 °C to 25 °C, which stayed as a transparent solution free of precipitates for 4.5 hours.

30 ml of a 20% solution of human serum albumin for infusion was added into a 250 ml infusion bag containing 180 ml normal saline solution (0.5% NaCl solution). After the albumin solution was mixed well with saline solution, the infusion bag was kept still at horizontal position, and 3 ml of the docetaxel API solution was injected rapidly into the infusion bag. Then the solution was mixed well to obtain a transparent solution free of precipitates. The pH of the obtained transparent solution is 6.97. The particulate matter of the transparent solution was also measured. 1 ml of the transparent solution has about 29 particles that are greater than 10 µm in size and about 0 particle that is greater than 25 µm in size. The transparent solution was kept at 20 °C to 25 °C, which stayed as a transparent solution free of precipitates for 3.5 hours.

### Example 11:

20.9 mg citric acid was dissolved in 20 ml dehydrated ethanol to obtain an citric acid solution. 100.3 mg of docetaxel was dissolved in 4.5 ml of dehydrated ethanol, followed by adding 0.5 ml of the prepared citric acid solution to obtain a docetaxel API solution. 9.5 ml of a 20% solution of human serum albumin for infusion was added into a 100 ml vial containing 60 ml normal saline solution (0.5% NaCl solution). After the albumin solution was mixed well with saline solution, the vial was kept still at horizontal position, and 1 ml of the docetaxel API solution was injected rapidly into the vial. Then the solution was mixed well to obtain a transparent solution free of precipitates. The pH of the obtained transparent solution is 6.96. The particulate matter of the transparent solution was also measured. 1 ml of the transparent solution has about 42 particles that are greater than 10 µm in size and about 1 particle that is greater than 25 µm in size. The transparent solution was kept at 20 °C to 25 °C, which stayed as a transparent solution free of precipitates for 4 hours.

9 ml of a 20% solution of human serum albumin for infusion was added into a 100 ml vial containing 60 ml normal saline solution (0.5% NaCl solution). After the albumin solution was mixed well with saline solution, the vial was kept still at horizontal position, and 1 ml of the docetaxel API solution was injected rapidly into the vial. Then the solution was mixed well to obtain a transparent solution free of precipitates. The pH of the obtained transparent solution is 6.96. The particulate matter of the transparent solution was also measured. 1 ml of the transparent solution has about 101 particles that are greater than 10 µm in size and about 3 particles that are greater than 25 µm in size. The transparent solution was kept at 20 °C to 25 °C, which stayed as a transparent solution free of precipitates for 2.5 hours.

8.5 ml of a 20% solution of human serum albumin for infusion was added into a 100 ml vial containing 60 ml normal saline solution (0.5% NaCl solution). After the albumin solution was mixed well with saline solution, the vial was kept still at horizontal position, and 1 ml of the docetaxel API solution was injected rapidly into the vial. Then the solution was mixed well to obtain a transparent solution free of precipitates. The pH of the obtained transparent solution is 6.95. The particulate matter of the transparent solution was also measured. 1 ml of the transparent solution has about 17 particles that are greater than 10 µm in size and about 1 particle that is greater than 25 µm in size. The transparent solution was kept at 20 °C to 25 °C, which stayed as a transparent solution free of precipitates for 2 hours.

### Example 12:

19.4 mg citric acid was dissolved in 20 ml dehydrated ethanol to obtain an citric acid solution. 200.5 mg of docetaxel was dissolved in 9.6 ml of dehydrated ethanol, followed by adding 0.4 ml of the prepared citric acid solution to obtain a docetaxel API solution. 25 ml of a 20% solution of human serum albumin for infusion was added into a 250ml infusion bag containing 150 ml normal saline solution (0.5% NaCl solution). After the albumin solution was mixed well with saline solution, the infusion bag was kept still at horizontal position, and 2.5 ml of the docetaxel API solution was injected rapidly into the infusion bag by injecting underneath the liquid surface of the albumin solution (the injection process took about 1-2 seconds). Then the infusion bag was immediately gently inverted for about 20-30 times, and the solution was mixed well to obtain a transparent solution free of precipitates. The pH of the obtained transparent solution is 6.98. The particulate matter of the transparent solution was also measured. 1 ml of the transparent solution has about 161 particles that are greater than 10 µm in size and about 13 particles that are greater than 25 µm in size. The solution was then divided into two portions. The one portion of the transparent solution was kept at 20 °C to 25 °C, which stayed as a transparent solution free of precipitates for 2 hours. The other portion of the transparent solution was kept at 2 °C to 8 °C for 4 hours, which stayed as a transparent solution free of precipitates. After 4 hours, the solution was kept at 20 °C to 25 °C, which stayed as a transparent solution free of precipitates for additional 1.5 hours.

17.5 ml of a 20% solution of human serum albumin for infusion was added into a 250 ml infusion bag containing 105 ml normal saline solution (0.5% NaCl solution). After the albumin solution was mixed well with saline solution, the infusion bag was kept still at horizontal position, and 1.75 ml of the docetaxel API solution was injected rapidly into the infusion bag by injecting underneath the liquid surface of the albumin solution (the injection process took about 2 seconds). Then the infusion bag was immediately gently inverted for about 20-30 times, and the solution was mixed well to obtain a transparent solution free of precipitates. The particulate matter of the transparent solution was measured. 1 ml of the transparent solution has about 81 particles that are greater than 10 µm in size and about 1 particle that is greater than 25 µm in size. The solution was then divided into two portions. The one portion of the transparent solution was kept at 20 °C to 25 °C, which stayed as a transparent solution free of precipitates for 5 hours. The other portion of the transparent solution was kept at 2 °C to 8 °C for 4 hours, which stayed as a transparent solution free of precipitates. After 4 hours, the solution was kept at 20 °C to 25 °C, which stayed as a transparent solution initially and became cloudy in about 0.5 hour.

### Example 13: (not according to the claims)

100.3 mg of docetaxel was dissolved in 5 ml of dehydrated ethanol to obtain a docetaxel API solution. After 35 ml of normal saline solution (0.5% NaCl solution) was added into a 50 ml vail, the vial was kept still at horizontal position, and 0.5 ml of the docetaxel API solution was injected rapidly into the vial by injecting underneath the liquid surface of the saline solution (the injection process took about 2 seconds). Then the vial was immediately gently inverted for about 10 times, and a transparent solution was obtained initially. The precipitations appeared in the solution in about 10 minutes.

5 ml of a 20% solution of human serum albumin for infusion was added into a 50 ml vial containing 30 ml normal saline solution (0.5% NaCl solution). After the albumin solution was mixed well with saline solution, the vial was kept still at horizontal position, and 0.5 ml of the docetaxel API solution was injected rapidly into the vial by injecting underneath the liquid surface of the albumin solution (the injection process took about 2 seconds). Then the vial was immediately gently inverted for about 10 times, and the solution was mixed well to obtain a transparent solution free of precipitates. The solution stayed as a transparent solution free of precipitates for 4.5 hours.

4 ml of a 20% solution of human serum albumin for infusion was added into a 50 ml vial containing 31 ml normal saline solution (0.5% NaCl solution). After the albumin solution was mixed well with saline solution, the vial was kept still at horizontal position, and 0.5 ml of the docetaxel API solution was injected rapidly into the vial by injecting underneath the liquid surface of the albumin solution (the injection process took about 2 seconds). Then the vial was immediately gently inverted for about 10 times, and the solution was mixed well to obtain a transparent solution free of precipitates. The solution stayed as a transparent solution free of precipitates for 4.5 hours.

2.5 ml of a 20% solution of human serum albumin for infusion was added into a 50 ml vial containing 32.5 ml normal saline solution (0.5% NaCl solution). After the albumin solution was mixed well with saline solution, the vial was kept still at horizontal position, and 0.5 ml of the docetaxel API solution was injected rapidly into the vial by injecting underneath the liquid surface of the albumin solution (the injection process took about 2 seconds). Then the vial was immediately gently inverted for about 10 times, and the solution was mixed well to obtain a transparent solution free of precipitates. The solution stayed as a transparent solution free of precipitates for 3.5 hours.

### Example 14: (not according to the claims)

100.3 mg of docetaxel was dissolved in 5 ml of dehydrated ethanol to obtain a docetaxel API solution. 15 ml of a 20% solution of human serum albumin for infusion was added into a 250 ml infusion bag containing 90 ml normal saline solution (0.5% NaCl solution). After the albumin solution was mixed well with saline solution, the infusion bag was kept still at horizontal position, and 1.5 ml of the docetaxel API solution was injected rapidly into the infusion bag by injecting underneath the liquid surface of the albumin solution (the injection process took about 3 seconds). Then the infusion bag was immediately gently inverted for about 20 times, and the solution was mixed well to obtain a transparent solution free of precipitates. The particulate matter of the transparent solution was measured. 1 ml of the transparent solution has about 78 particles that are greater than 10 µm in size and about 3 particles that are greater than 25 µm in size. The solution stayed as a transparent solution free of precipitates for 3 hours.

12 ml of a 20% solution of human serum albumin for infusion was added into a 250 ml infusion bag containing 93 ml normal saline solution (0.5% NaCl solution). After the albumin solution was mixed well with saline solution, the infusion bag was kept still at horizontal position, and 1.5 ml of the docetaxel API solution was injected rapidly into the infusion bag by injecting underneath the liquid surface of the albumin solution (the injection process took about 3 seconds). Then the infusion bag was immediately gently inverted for about 20 times, and the solution was mixed well to obtain a transparent solution free of precipitates. The particulate matter of the transparent solution was measured. 1 ml of the transparent solution has about 90 particles that are greater than 10 µm in size and about 4 particles that are greater than 25 µm in size. The solution stayed as a transparent solution free of precipitates for 3.5 hours.

7.5 ml of a 20% solution of human serum albumin for infusion was added into a 250 ml infusion bag containing 97.5 ml normal saline solution (0.5% NaCl solution). After the albumin solution was mixed well with saline solution, the infusion bag was kept still at horizontal position, and 1.5 ml of the docetaxel API solution was injected rapidly into the infusion bag by injecting underneath the liquid surface of the albumin solution (the injection process took about 3 seconds). Then the infusion bag was immediately gently inverted for about 20 times, and the solution was mixed well to obtain a transparent solution free of precipitates. The particulate matter of the transparent solution was measured. 1 ml of the transparent solution has about 63 particles that are greater than 10 µm in size and about 2 particles that are greater than 25 µm in size. The solution stayed as a transparent solution free of precipitates for 3 hours.

### Example 15:

20.1 mg citric acid was dissolved in 20 ml dehydrated ethanol to obtain a citric acid solution. 300.7 mg of docetaxel was dissolved in 14.4 ml of dehydrated ethanol, followed by adding 0.6 ml of the prepared citric acid solution to obtain a docetaxel API solution. 7.5 ml of a 20% solution of human serum albumin for infusion was added into a 250 ml infusion bottle containing 97.5 ml normal saline solution (0.5% NaCl solution). After the albumin solution was mixed well with saline solution, the infusion bottle was kept still at horizontal position, and 1.5 ml of the docetaxel API solution was injected rapidly into the infusion bottle by injecting underneath the liquid surface of the albumin solution (the injection process took about 2 seconds). Then the infusion bottle was immediately gently inverted for about 20 times, and the solution was mixed well to obtain a transparent solution free of precipitates. The particulate matter of the transparent solution was measured. 1 ml of the transparent solution has about 88 particles that are greater than 10 µm in size and about 8 particles that are greater than 25 µm in size. The solution stayed as a transparent solution free of precipitates for 2.5 hours.

6 ml of a 20% solution of human serum albumin for infusion was added into a 250 ml infusion bottle containing 99 ml normal saline solution (0.5% NaCl solution). After the albumin solution was mixed well with saline solution, the infusion bottle was kept still at horizontal position, and 1.5 ml of the docetaxel API solution was injected rapidly into the infusion bottle by injecting underneath the liquid surface of the albumin solution (the injection process took about 2 seconds). Then the infusion bottle was immediately gently inverted for about 20 times, and the solution was mixed well to obtain a transparent solution free of precipitates. The particulate matter of the transparent solution was measured. 1 ml of the transparent solution has about 37 particles that are greater than 10 µm in size and about 0 particle that is greater than 25 µm in size. The solution stayed as a transparent solution free of precipitates for 3 hours.

4.5 ml of a 20% solution of human serum albumin for infusion was added into a 250 ml infusion bottle containing 100.5 ml normal saline solution (0.5% NaCl solution). After the albumin solution was mixed well with saline solution, the infusion bottle was kept still at horizontal position, and 1.5 ml of the docetaxel API solution was injected rapidly into the infusion bottle by injecting underneath the liquid surface of the albumin solution (the injection process took about 2 seconds). Then the infusion bottle was immediately gently inverted for about 20 times, and the solution was mixed well to obtain a transparent solution free of precipitates. The particulate matter of the transparent solution was measured. 1 ml of the transparent solution has about 65 particles that are greater than 10 µm in size and about 2 particles that are greater than 25 µm in size. The solution stayed as a transparent solution free of precipitates for 3 hours.

30 ml of a 20% solution of human serum albumin for infusion was added into a 500 ml infusion bottle containing 390 ml normal saline solution (0.5% NaCl solution). After the albumin solution was mixed well with saline solution, the infusion bottle was kept still at horizontal position, and 6 ml of the docetaxel API solution was injected rapidly into the infusion bottle by injecting underneath the liquid surface of the albumin solution (the injection process took about 2 seconds). Then the infusion bottle was immediately gently inverted for about 20-30 times, and the solution was mixed well to obtain a transparent solution free of precipitates. The particulate matter of the transparent solution was measured. 1 ml of the transparent solution has about 60 particles that are greater than 10 µm in size and about 4 particles that are greater than 25 µm in size. The solution was then divided into three portions. The one portion of the transparent solution was kept at 20 °C to 25 °C, which stayed as a transparent solution free of precipitates for 5.5 hours. The second portion of the transparent solution was kept at 2 °C to 8 °C, which stayed as a transparent solution free of precipitates for 7 hours. The third portion of the transparent solution was kept at 2 °C to 8 °C for 5 hours, which stayed as a transparent solution free of precipitates. After 5 hours, the solution was kept at 20 °C to 25 °C, which stayed as a transparent solution free of precipitates for additional 1.5 hours.

### Example 16:

44.84 g of docetaxel was weighed and dissolved in 671.98 g of dehydrated ethanol in a 2 liter beaker, and then docetaxel solution was transferred to a 5 liter beaker. The 2 liter beaker was rinsed by 822.63 g of dehydrated ethanol in 3 portions and the rinsed ethanol was also transferred into the 5 liter beaker. 87.7 mg of citric acid was dissolved in 42.05 g of dehydrated ethanol in a 100 ml beaker. Then the citric acid solution was transferred into the 5 liter beaker when the docetaxel solution in the 5 liter beaker was stirred. The 100 ml beaker was then rinsed by 92.56 g of dehydrated ethanol in 3 portions and the rinsed ethanol was also transferred into the 5 liter beaker. The resulting docetaxel solution in 5 liter beaker was stirred for additional about 1 minute, and followed by filtered through a 0.22µm filter. The filtered docetaxel solution was filled into 10 ml glass vials using an automated filling machine. Total a batch of 470 vials with 4.4ml docetaxel solution (10% over-fill) in 10ml vial were obtained. The stability test of the obtained docetaxel solution (docetaxel product) were tested under long term condition (2~8°C) and under accelerated condition (25±2°C/60%±5%RH). The stability test results are shown in the **Table 1** and **Table 2.** The stability test data indicate that the docetaxel solution obtained was stable under long term condition and accelerated condition.

**Table 1 Stability Test Results at 2-8°C**

| Test | Test Time Point (Month) | | |
|---|---|---|---|
| | 0 | 3 | 6 |
| Assay (mg/ml) | 20.5 | 20.48 | 20.48 |
| Total Impurities (%) | 0.12 | 0.16 | 0.12 |
| pH | 4.4 | 4.5 | 4.4 |

**Table 2 Stability Test Results at 25±2°C/60%±5%RH**

| Test | Test Time Point (Month) | | | | |
|---|---|---|---|---|---|
| | 0 | 1 | 2 | 3 | 6 |
| Assay (mg/ml) | 20.5 | 20.64 | 20.56 | 20.38 | 20.52 |
| Total Impurities (%) | 0.12 | 0.22 | 0.16 | 0.21 | 0.53 |
| pH | 4.4 | 4.4 | 4.3 | 4.5 | 4.4 |

### Example 17:

4 different formulations of docetaxel solution were prepared by dissolving docetaxel and citric acid in dehydrated ethanol. The compositions of 4 formulations are listed as below: Formulation A (20mg/ml docetaxel and 0.01mg/ml citric acid), Formulation B (20mg/ml docetaxel and 0.02mg/ml citric acid), Formulation C (20mg/ml docetaxel and 0.1mg/ml citric acid), and Formulation D (20mg/ml docetaxel and 0.2mg/ml citric acid). The pH value of 4 formulations of docetaxel solution along with the docetaxel solution obtained in example 16 (Formulation E: 20mg/ml docetaxel and 0.04mg/ml citric acid) were measured. The results of the pH test are shown in the **Table 3.**

**Table 3 pH values of Formulations of docetaxel solution**

| Formulation | A | B | C | D | E |
|---|---|---|---|---|---|
| Weight ratio of docetaxel and citric acid | 2000:1 | 1000:1 | 200:1 | 100:1 | 500:1 |
| pH | 4.86 | 4.5 | 3.95 | 3.72 | 4.36 |

### Example 18:

A batch of Docetaxel Injection (88mg/4.4mL) with docetaxel (88mg) and citric acid (0. 176mg) in dehydrated ethanol (4.4mL) was obtained following the same manufacturing process and procedure described in Example 16. The reconstituted parenteral infusion solution of Docetaxel (160ml) was prepared following the procedure below. 15 mL of 20% human Albumin infusion solution is mixed with 143 mL of 0.9% sodium chloride for injection in an infusion bottle first, then followed by injecting 2mL of Docetaxel injection (40mg/docetaxel) rapidly into the Albumin saline solution in the infusion bottle and mixed well with the Albumin saline solution to obtain an infusion solution of Docetaxel as a clear solution. The obtained infusion solution of Docetaxel was tested for particle size distribution using Dynamic Light Scattering (DLS). For comparison, an Albumin saline solution was also tested for particle size distribution using DLS. For preparation of Albumin saline solution, the same preparation procedure was used as for preparation of the infusion solution of Docetaxel, except that same amount (2mL) of anhydrous ethanol was used (injected) instead of 2mL of Docetaxel Injection in the last step of the preparation. The DLS test results of Docetaxel infusion solution and Albumin saline solution are provided in Figure 1. Docetaxel infusion solution has almost identical particle size distribution profile compared to Albumin saline solution. The DLS data demonstrated that Docetaxel infusion solution is a simple solution same as Albumin saline solution. See Figures 1A and 1B.

### Example 19:

Percentage of free (unbound) docetaxel in reconstituted Docetaxel infusion solution prepared using Docetaxel Injection (88mg/4.4mL) obtained in Example 16 was tested. The Docetaxel infusion solution was prepared using the same procedure described in Example 18. Percentage of free (unbound) docetaxel in Docetaxel infusion solution is measured by ultrafiltration through a 30-kDa membrane. Percentage of free (unbound) docetaxel in Docetaxel infusion solution is in the range of 62.8% - 67%. The results of percentage of free (unbound) docetaxel in reconstituted Docetaxel infusion solution are provided in **Table 4.**

**Table 4 Free (Unbound) Docetaxel and Albumin Bound Docetaxel in Reconstituted Docetaxel infusion Solution**

| **Test Solution** | **Free Docetaxel** | **Albumin Bound Docetaxel** | **Average** | |
|---|---|---|---|---|
| | | | **Free Docetaxel** | **Albumin Bound Docetaxel** |
| Docetaxel Infusion Solution | 67.0% | 33.0% | 64.5% | 35.5% |
| | 62.8% | 37.2% | | |
| | 63.6% | 36.4% | | |

### Example 20:

Percentages of free (unbound) docetaxel in two reconstituted Docetaxel infusion solutions were studies by varying the amount of human serum Albumin in Docetaxel infusion solution. Docetaxel infusion solution-A with less amount of Albumin and Docetaxel infusion solution-B with more amount of Albumin relative to the Docetaxel infusion solution in Example 19 were prepared.

Preparation of Docetaxel infusion solution-A: 5 mL of 20% human Albumin infusion solution is mixed with 74 mL of 0.9% sodium chloride for injection in an infusion bottle first, then followed by injecting 1mL(20mg) of Docetaxel injection (prepared in Example 16) rapidly into the Albumin saline solution in the infusion bottle and mixed well with the Albumin saline solution to obtain Docetaxel infusion solution-A as a clear solution.

Preparation of Docetaxel infusion solution-B: 10 mL of 20% human Albumin infusion solution is mixed with 69 mL of 0.9% sodium chloride for injection in an infusion bottle first, then followed by injecting 1mL(20mg) of Docetaxel injection (prepared in Example 16) rapidly into the Albumin saline solution in the infusion bottle and mixed well with the Albumin saline solution to obtain Docetaxel infusion solution-B as a clear solution.

Percentages of free (unbound) docetaxel in Docetaxel infusion solution-A and Docetaxel infusion solution-B are measured by ultrafiltration through a 30-kDa membrane. The results of percentage of free (unbound) docetaxel in reconstituted Docetaxel infusion solutions are provided in **Table 5.**

**Table 5 Free (Unbound) Docetaxel and Albumin Bound Docetaxel in Reconstituted Docetaxel infusion Solution-A and -B**

| **Test Solution** | **Free Docetaxel** | **Albumin Bound Docetaxel** |
|---|---|---|
| Docetaxel Infusion Solution-A | 66.8% | 33.2% |
| Docetaxel Infusion Solution-B | 61.2% | 38.8% |

### Example 21:

10 mL of 20% human Albumin infusion solution is mixed with 148 mL of 0.9% sodium chloride for injection in a 250ml infusion bottle first, then followed by injecting 2mL(40mg) of Docetaxel injection (prepared in Example 16) rapidly into the Albumin saline solution in the infusion bottle, inverted the infusion bottle and mixed well with the Albumin saline solution to obtain a clear infusion solution. The infusion solution stayed transparent free of precipitates for 6 hours.

### Example 22: (not according to the claims)

Prepartion of Docetaxel injection containing polysorbate 80: 201.5mg of docetaxel was dissovled in 5mL anhydrous ethanol, followed by adding 5mL polysorbate 80 into the solution to obtain a Docetaxel injection formulated with polysorbate 80 as a clear solution. 295.5 mL of 0.9% sodium chloride for injection was prepared in a 500ml infusion bottle first, then followed by injecting 4.5mLof Docetaxel injection formulated by polysorbate 80 and ethanol (1:1, v/v) (prepared above) rapidly into the saline solution in the infusion bottle, inverted the infusion bottle and mixed well with the saline solution to obtain a clear infusion solution. The infusion solution stayed transparent free of precipitates for 6 hours.

### Example 23:

A batch of Docetaxel Injection (88mg/4.4mL) with docetaxel (88mg) and citric acid (0.176mg) in dehydrated ethanol (4.4mL) was obtained following the same manufacturing process and procedure described in Example 16.

37.5 mL of 20% human Albumin infusion solution is mixed with 357.5 mL of 0.9% sodium chloride for injection in a 500ml infusion bottle first, then followed by injecting 5mL of the Docetaxel injection (prepared above) rapidly into the Albumin saline solution in the infusion bottle, while keeping the infusion bottle still. After the infusion bottle was inverted for about 20 times and the solution was mixed well, a clear infusion solution was obtained. The infusion solution stayed transparent free of precipitates for 7 hours.

22.5 mL of 20% human Albumin infusion solution is mixed with 214.5 mL of 0.9% sodium chloride for injection in a 250ml infusion bottle first, then followed by injecting 3mL of the Docetaxel injection (prepared above) rapidly into the Albumin saline solution in the infusion bottle, while keeping the infusion bottle still. After the infusion bottle was inverted for about 30 times and the solution was mixed well, a clear infusion solution was obtained. The infusion solution stayed transparent free of precipitates for 8 hours.

## Claims

1. A pharmaceutical formulation of docetaxel comprising two compositions that are mixed prior to being infused or administered to patients, that comprises: (a) a first liquid composition comprising docetaxel, or a pharmaceutically acceptable salt thereof, ethanol, and an organic acid, wherein the organic acid is not aspartic acid or glutamic acid, and (b) a second aqueous composition comprising human serum albumin and a parenterally acceptable vehicle, wherein the pharmaceutical formulation does not contain Polysorbate 80, and wherein the two compositions are mixed in less than 24 hours prior to being infused or administered to patients.

2. The pharmaceutical formulation of claim 1, wherein the mixing of the two compositions is done in an infusion bag or bottle, and/or wherein the first liquid composition is injected into an infusion bag or bottle which contains the second aqueous composition.

3. The pharmaceutical formulation of claim 1 or claim 2, wherein the first liquid composition is a sterile solution.

4. The pharmaceutical formulation of any one of claims 1-3, wherein the concentration of docetaxel, or a pharmaceutically acceptable salt thereof, in the formulation after mixing of the first liquid composition and the second aqueous composition is from about 0.05 mg/ml to about 1 mg/ml.

5. The pharmaceutical formulation of any one of claims 1-4, wherein the organic acid is selected from the group consisting of citric acid, acetic acid, formic acid ascorbic acid, benzoic acid, tartaric acid, lactic acid, maleic acid, succinic acid, and any pharmaceutically acceptable salt thereof.

6. The pharmaceutical formulation of any one of claims 1-5, wherein the second aqueous composition is prepared by adding a human serum albumin solution into a parenterally acceptable vehicle, optionally wherein the concentration of human serum albumin in the second aqueous composition is from about 0.1% to about 20% (w/v).

7. A transparent parenteral infusion composition of docetaxel, or a pharmaceutically acceptable salt thereof, free of precipitates, the composition comprising human serum albumin, an organic acid, wherein the organic acid is not aspartic acid or glutamic acid, and said docetaxel, or a pharmaceutically acceptable salt thereof, in a concentration from about 0.05 mg/ml to about 1 mg/ml of docetaxel in a parenterally acceptable vehicle, wherein the said infusion solution is obtained by injecting a first liquid composition comprising docetaxel, or a pharmaceutically acceptable salt thereof, the organic acid, and ethanol into an infusion bag or bottle containing a second aqueous composition comprising human serum albumin in a parenterally acceptable vehicle, wherein mixing or agitation is not needed in the injection process, and wherein the said infusion solution does not contain Polysorbate 80.

8. The parenteral infusion composition of docetaxel of claim 7, wherein the parenterally acceptable vehicle is a normal saline solution or dextrose solution.

9. The parenteral infusion composition of docetaxel of claim 7 or claim 8, wherein the amount of ethanol in the parenteral infusion composition of docetaxel, or a pharmaceutically acceptable salt thereof, is no more than 10% (v/v), optionally wherein the amount of ethanol in the parenteral infusion composition of docetaxel, or a pharmaceutically acceptable salt thereof, is no more than 2% (v/v).

10. The parenteral infusion composition of docetaxel of any one of claims 7-9, wherein the parenteral infusion composition of docetaxel, or a pharmaceutically acceptable salt thereof, is prepared less than about 24 hours prior to being infused or administered to patients.

11. The parenteral infusion composition of docetaxel of any one of claims 7-10, wherein the pH value of the parenteral infusion composition is from about 4 to about 9.5.

12. The parenteral infusion composition of docetaxel of any one of claims 7-11, wherein 1ml of the parenteral infusion composition comprises no more than 100 particles that are greater than 10 µm in size, measured using Dynamic Light Scattering (DLS), and/or
wherein the transparent infusion solution stays transparent free of precipitates for at least 4 hours.

13. The parenteral infusion composition of docetaxel of any one of claims 7-12, wherein weight ratio of human serum albumin to docetaxel, or a pharmaceutically acceptable salt thereof, is from about 20:1 to about 200:1.

14. The parenteral infusion composition of docetaxel of any one of claims 7-13, wherein the time of injecting the first liquid composition into the infusion bag or bottle containing the second aqueous composition is no more than 15 seconds, and/or wherein the injection of the first liquid composition into the infusion bag or bottle containing the second aqueous composition is done in the temperature from about 15 °C to about 30 °C.

15. The pharmaceutical formulation of docetaxel of any one of claims 1-6 for use in a method of treating cancer, the method comprising administering to a subject in need thereof a therapeutically effective amount of said pharmaceutical formulation, wherein the cancer is selected from the group consisting of bladder cancer, brain cancer, breast cancer, colorectal cancer, cervical cancer, gastrointestinal cancer, genitourinary cancer, head and neck cancer, lung cancer, ovarian cancer, pancreatic cancer, prostate cancer, renal cancer, skin cancer, and testicular cancer.

## Patentansprüche

1. Pharmazeutische Formulierung von Docetaxel, die zwei Zusammensetzungen umfasst, die vor der Infusion oder Verabreichung an Patienten gemischt werden, die Folgendes umfasst: (a) eine erste flüssige Zusammensetzung, die Docetaxel oder ein pharmazeutisch unbedenkliches Salz davon, Ethanol und eine organische Säure umfasst, wobei die organische Säure weder Asparaginsäure noch Glutaminsäure ist, und (b) eine zweite wässrige Zusammensetzung, die Humanserumalbumin und ein parenteral unbedenkliches Vehikel umfasst, wobei die pharmazeutische Formulierung kein Polysorbat 80 enthält und wobei die zwei Zusammensetzungen weniger als 24 Stunden vor der Infusion oder Verabreichung an Patienten gemischt werden.

2. Pharmazeutische Formulierung nach Anspruch 1, wobei das Mischen der beiden Zusammensetzungen in einem Infusionsbeutel oder einer Infusionsflasche erfolgt und/oder wobei die erste flüssige Zusammensetzung in einen Infusionsbeutel oder eine Infusionsflasche injiziert wird, die die zweite wässrige Zusammensetzung enthält.

3. Pharmazeutische Formulierung nach Anspruch 1 oder Anspruch 2, wobei die erste flüssige Zusammensetzung eine sterile Lösung ist.

4. Pharmazeutische Formulierung nach einem der Ansprüche 1-3, wobei die Konzentration von Docetaxel oder einem pharmazeutisch unbedenklichen Salz davon in der Formulierung nach dem Mischen der ersten flüssigen Zusammensetzung und der zweiten wässrigen Zusammensetzung etwa 0,05 mg/ml bis etwa 1 mg/ml beträgt.

5. Pharmazeutische Formulierung nach einem der Ansprüche 1-4, wobei die organische Säure aus der aus Citronensäure, Essigsäure, Ameisensäure, Ascorbinsäure, Benzoesäure, Weinsäure, Milchsäure, Maleinsäure, Bernsteinsäure und einem beliebigen pharmazeutisch unbedenklichen Salz davon bestehenden Gruppe ausgewählt ist.

6. Pharmazeutische Formulierung nach einem der Ansprüche 1-5, wobei die zweite wässrige Zusammensetzung durch Zugabe einer Lösung von Humanserumalbumin zu einem parenteral unbedenklichen Vehikel hergestellt wird, wobei die Konzentration an Humanserumalbumin in der zweiten wässrigen Zusammensetzung gegebenenfalls etwa 0,1 % bis etwa 20 % (w/v) beträgt.

7. Transparente parenterale Infusionszusammensetzung von Docetaxel oder einem pharmazeutisch unbedenklichen Salz davon, frei von Präzipitaten, wobei die Zusammensetzung Humanserumalbumin, eine organische Säure, wobei die organische Säure weder Asparaginsäure noch Glutaminsäure ist, und das Docetaxel oder ein pharmazeutisch unbedenkliches Salz davon in einer Konzentration von etwa 0,05 mg/ml bis etwa 1 mg/ml Docetaxel in einem parenteral unbedenklichen Vehikel umfasst, wobei die Infusionslösung durch Injektion einer ersten flüssigen, Docetaxel oder ein pharmazeutisch unbedenkliches Salz davon, die organische Säure und Ethanol umfassenden Zusammensetzung in einen Infusionsbeutel oder eine Infusionsflasche, der/die eine zweite wässrige Zusammensetzung enthält, die Humanserumalbumin in einem parenteral unbedenklichen Vehikel umfasst, erhalten wird, wobei Mischen oder Rühren beim Injektionsvorgang nicht erforderlich ist und wobei die Infusionslösung kein Polysorbat 80 enthält.

8. Parenterale Infusionszusammensetzung von Docetaxel nach Anspruch 7, wobei das parenteral unbedenkliche Vehikel eine normale Kochsalzlösung oder Dextroselösung ist.

9. Parenterale Infusionszusammensetzung von Docetaxel nach Anspruch 7 oder Anspruch 8, wobei die Menge an Ethanol in der parenteralen Infusionszusammensetzung von Docetaxel oder einem pharmazeutisch unbedenklichen Salz davon nicht mehr als 10 Vol.-% beträgt, wobei die Menge an Ethanol in der parenteralen Infusionszusammensetzung von Docetaxel oder einem pharmazeutisch unbedenklichen Salz davon nicht mehr als 2 Vol.-% beträgt.

10. Parenterale Infusionszusammensetzung von Docetaxel nach einem der Ansprüche 7-9, wobei die parenterale Infusionszusammensetzung von Docetaxel oder einem pharmazeutisch unbedenklichen Salz davon weniger als etwa 24 Stunden vor der Infusion oder Verabreichung an Patienten hergestellt wird.

11. Parenterale Infusionszusammensetzung von Docetaxel nach einem der Ansprüche 7-10, wobei der pH-Wert der parenteralen Infusionszusammensetzung etwa 4 bis etwa 9,5 beträgt.

12. Parenterale Infusionszusammensetzung von Docetaxel nach einem der Ansprüche 7-11, wobei 1 ml der parenteralen Infusionszusammensetzung nicht mehr als 100 Partikel mit einer Größe von mehr als 10 µm umfasst, gemessen durch dynamische Lichtstreuung (Dynamic Light Scattering, DLS), und/oder wobei die transparente Infusionslösung mindestens 4 Stunden transparent und frei von Präzipitaten bleibt.

13. Parenterale Infusionszusammensetzung von Docetaxel nach einem der Ansprüche 7-12, wobei das Gewichtsverhältnis von Humanserumalbumin zu Docetaxel oder einem pharmazeutisch unbedenklichen Salz davon etwa 20:1 bis etwa 200:1 beträgt.

14. Parenterale Infusionszusammensetzung nach Docetaxel nach einem der Ansprüche 7-13, wobei die Zeit des Injizierens der ersten flüssigen Zusammensetzung in den Infusionsbeutel oder die Infusionsflasche, der/die die zweite wässrige Zusammensetzung enthält, nicht mehr als 15 Sekunden beträgt und/oder wobei das Injizieren der ersten flüssigen Zusammensetzung in den Infusionsbeutel oder die Infusionsflasche, der/die die zweite wässrige Zusammensetzung enthält, bei einer Temperatur von etwa 15 °C bis etwa 30 °C erfolgt.

15. Pharmazeutische Formulierung von Docetaxel nach einem der Ansprüche 1-6 zur Verwendung in einem Verfahren zur Behandlung von Krebs, wobei das Verfahren die Verabreichung einer therapeutisch wirksamen Menge der pharmazeutischen Formulierung an ein dessen bedürftiges Subjekt umfasst, wobei der Krebs aus der aus Blasenkrebs, Hirnkrebs, Brustkrebs, Kolorektalkrebs, Gebärmutterhalskrebs, Magen-Darm-Krebs, Urogenitalkrebs, Kopf- und Halskrebs, Lungenkrebs, Eierstockkrebs, Bauchspeicheldrüsenkrebs, Prostatakrebs, Nierenkrebs, Hautkrebs und Hodenkrebs bestehenden Gruppe ausgewählt ist.

## Revendications

1. Formulation pharmaceutique de docétaxel comprenant deux compositions qui sont mélangées avant d'être perfusées ou administrées à des patients, qui comprend : (a) une première composition liquide comprenant du docétaxel, ou un sel pharmaceutiquement acceptable de celui-ci, de l'éthanol et un acide organique, dans laquelle l'acide organique n'est pas l'acide aspartique ou l'acide glutamique, et (b) une deuxième composition aqueuse comprenant de la sérum-albumine humaine et un véhicule parentéralement acceptable, dans laquelle la formulation pharmaceutique ne contient pas de Polysorbate 80, et dans laquelle les deux compositions sont mélangées en moins de 24 heures avant d'être perfusées ou administrées à des patients.

2. Formulation pharmaceutique selon la revendication 1, dans laquelle le mélange des deux compositions est effectué dans une poche ou une bouteille de perfusion, et/ou dans laquelle la première composition liquide est injectée dans une poche ou une bouteille de perfusion qui contient la deuxième composition aqueuse.

3. Formulation pharmaceutique selon la revendication 1 ou la revendication 2, dans laquelle la première composition liquide est une solution stérile.

4. Formulation pharmaceutique selon l'une quelconque des revendications 1 à 3, dans laquelle la concentration de docétaxel, ou d'un sel pharmaceutiquement acceptable de celui-ci, dans la formulation après mélange de la première composition liquide et de la deuxième composition aqueuse est d'environ 0,05 mg/ml à environ 1 mg/ml.

5. Formulation pharmaceutique selon l'une quelconque des revendications 1 à 4, dans laquelle l'acide organique est choisi dans le groupe constitué par l'acide citrique, l'acide acétique, l'acide formique, l'acide ascorbique, l'acide benzoïque, l'acide tartrique, l'acide lactique, l'acide maléique, l'acide succinique et tout sel pharmaceutiquement acceptable de celui-ci.

6. Formulation pharmaceutique selon l'une quelconque des revendications 1 à 5, dans laquelle la deuxième composition aqueuse est préparée en ajoutant une solution de sérum-albumine humaine dans un véhicule parentéralement acceptable, éventuellement dans laquelle la concentration de sérum-albumine humaine dans la deuxième composition aqueuse est d'environ 0,1 % à environ 20 % (p/v).

7. Composition transparente de docétaxel pour perfusion parentérale, ou d'un sel pharmaceutiquement acceptable de celui-ci, exempte de précipités, la composition comprenant de la sérum-albumine humaine, un acide organique, dans laquelle l'acide organique n'est pas l'acide aspartique ou l'acide glutamique, et ledit docétaxel, ou un sel pharmaceutiquement acceptable de celui-ci, en une concentration d'environ 0,05 mg/ml à environ 1 mg/ml de docétaxel dans un véhicule parentéralement acceptable, dans laquelle ladite solution pour perfusion est obtenue par injection d'une première composition liquide comprenant du docétaxel, ou un sel pharmaceutiquement acceptable de celui-ci, l'acide organique et l'éthanol dans une poche ou bouteille de perfusion contenant une deuxième composition aqueuse comprenant de la sérum-albumine humaine dans un véhicule parentéralement acceptable, dans laquelle un mélange ou une agitation n'est pas nécessaire dans le processus d'injection, et dans lequel ladite solution de perfusion ne contient pas de Polysorbate 80.

8. Composition de docétaxel pour perfusion parentérale selon la revendication 7, dans laquelle le véhicule parentéralement acceptable est une solution saline normale ou une solution de dextrose.

9. Composition de docétaxel pour perfusion parentérale selon la revendication 7 ou la revendication 8, dans laquelle la quantité d'éthanol dans la composition de docétaxel pour perfusion parentérale, ou un sel pharmaceutiquement acceptable de celui-ci, n'est pas supérieure à 10 % (v/v), éventuellement dans laquelle la quantité d'éthanol dans la composition de docétaxel pour perfusion parentérale, ou un sel pharmaceutiquement acceptable de celui-ci, n'est pas supérieure à 2 % (v/v).

10. Composition de docétaxel pour perfusion parentérale selon l'une quelconque des revendications 7 à 9, dans laquelle la composition de docétaxel pour perfusion parentérale, ou un sel pharmaceutiquement acceptable de celui-ci, est préparée moins d'environ 24 heures avant d'être perfusée ou administrée à des patients.

11. Composition de docétaxel pour perfusion parentérale selon l'une quelconque des revendications 7 à 10, dans laquelle la valeur de pH de la composition pour perfusion parentérale est d'environ 4 à environ 9,5.

12. Composition de docétaxel pour perfusion parentérale selon l'une quelconque des revendications 7 à 11, dans laquelle 1 ml de la composition pour perfusion parentérale ne comprend pas plus de 100 particules qui ont une taille supérieure à 10 µm, mesurée en utilisant la diffusion dynamique de lumière (DLS), et/ou dans laquelle la solution pour perfusion transparente reste transparente exempte de précipités pendant au moins 4 heures.

13. Composition de docétaxel pour perfusion parentérale selon l'une quelconque des revendications 7 à 12, dans laquelle le rapport pondéral de la sérum-albumine humaine sur le docétaxel, ou un sel pharmaceutiquement acceptable de celui-ci, est d'environ 20:1 à environ 200:1.

14. Composition de docétaxel pour perfusion parentérale selon l'une quelconque des revendications 7 à 13, dans laquelle la durée d'injection de la première composition liquide dans la poche ou bouteille de perfusion contenant la deuxième composition aqueuse n'est pas supérieure à 15 secondes, et/ou dans laquelle l'injection de la première composition liquide dans la poche ou la bouteille de perfusion contenant la deuxième composition aqueuse est effectuée à une température d'environ 15 °C à environ 30 °C.

15. Formulation pharmaceutique de docétaxel selon l'une quelconque des revendications 1 à 6 pour une utilisation dans un procédé de traitement d'un cancer, le procédé comprenant l'administration à un sujet qui en a besoin d'une quantité thérapeutiquement efficace de ladite formulation pharmaceutique, dans laquelle le cancer est choisi dans le groupe constitué par le cancer de la vessie, le cancer du cerveau, le cancer du sein, le cancer colorectal, le cancer du col de l'utérus, le cancer gastro-intestinal, le cancer génito-urinaire, le cancer de la tête et du cou, le cancer du poumon, le cancer de l'ovaire, le cancer du pancréas, le cancer de la prostate, le cancer du rein, le cancer de la peau et le cancer testiculaire.
